# EUROPÄISCHE PATENTANMELDUNG

(11) **EP 1 561 809 A2**
(43) Veröffentlichungstag der Anmeldung: **10.08.2005**
(21) Anmeldenummer: 04001504.2
(22) Anmeldetag: 23.01.2004
(51) Int. Cl.: C12N 1/18, C12N 1/19, C12N 15/81, C12P 21/02, C07K 14/195, C07K 16/12, C07K 16/14

(54) **Phycocyanin exprimierende Eukaryotenzelle**

(71) Anmelder: Signalomics GmbH, 48565 Steinfurt (DE)
(72) Erfinder: Arntz, Claudia, Dr., 49525 Lengerich (DE); Block, Christoph, Dr., 48151 Münster (DE); Meinders, Danielle, Dipl.-Ing., 48565 Steinfurt (DE); Mittmann, Karin, Prof. Dr., 48151 Münster (DE)
(74) Vertreter: Von Renesse, Dorothea, Dr.

(57) **Zusammenfassung**

Die Erfindung betrifft eine rekombinante Eukaryontenzelle synthetisierend funktionell aktives Phycocyanin oder funktionell aktive Teile daraus.

SEQUENCE LISTING

<110>  Signalomics GmbH

<120>  Phycocyanin exprimierende Eukaryontenzelle

<130>  45 272 K

<140>  EP 04 001 504.2
<141>  2004-01-23

<160>  14

<170>  PatentIn version 3.3

<210>  1
<211>  191
<212>  DNA
<213>  synthetisch

<400>  1
aagcttccat ggaatctcga gacaccatca ccatcaccat taaggtatat aactctgtag      60

aaataaagag tatcatcttt caaaccgcgg attgtcgcga tcaaatcgat atgtcttatg     120

cggccgctta cgaccgttaa cttgttctag attgggctag cgttgagatc tttagaaacg     180

tcgacgaatt c                                                          191


<210>  2
<211>  191
<212>  DNA
<213>  synthetisch

<400>  2
aagctttcat gaaatctcga gacaccatca ccatcaccat taaggtatat aactctgtag      60

aaataaagag tatcatcttt caaaccgcgg attgtcgcga tcaaatcgat atgtcttatg     120

cggccgctta cgaccgttaa cttgttctag attgggctag cgttgagatc tttagaaacg     180

tcgacgaatt c                                                          191


<210>  3
<211>  173
<212>  DNA
<213>  synthetisch

<400>  3
aagcttccat ggaatctcga gataaggtat ataactctgt agaaataaag agtatcatct      60

ttcaaaccgc ggattgtcgc gatcaaatcg atatgtctta tgcggccgct tacgaccgtt     120

aacttgttct agattgggct agcgttgaga tctttagaaa cgtcgacgaa ttc           173


<210>  4
<211>  173
<212>  DNA
<213>  synthetisch

<400>  4
aagctttcat gaaatctcga gataaggtat ataactctgt agaaataaag agtatcatct      60

ttcaaaccgc ggattgtcgc gatcaaatcg atatgtctta tgcggccgct tacgaccgtt     120

aacttgttct agattgggct agcgttgaga tctttagaaa cgtcgacgaa ttc 173

<210> 5
<211> 731
<212> DNA
<213> S. cerevisiae Y190

<400> 5
gagctcatat ccttttgttg tttccgggtg tacaatatgg acttcctctt ttctggcaac 60

caaacccata catcgggatt cctataatac cttcgttggt ctccctaaca tgtaggtggc 120

ggaggggaga tatacaatag aacagatacc agacaagaca taatgggcta aacaagacta 180

caccaattac actgcctcat tgatggtggt acataacgaa ctaatactgt agccctagac 240

ttgatagcca tcatcatatc gaagtttcac tacccttttt ccatttgcca tctattgaag 300

taataatagg cgcatgcaac ttcttttctt tttttttctt ttctctctcc cccgttgttg 360

tctcaccata tccgcaatga caaaaaaatg atggaagaca ctaaaggaaa aaattaacga 420

caaagacagc accaacagat gtcgttgttc cagagctgat gaggggtatc tcgaagcaca 480

cgaaactttt tccttccttc attcacgcac actactctct aatgagcaac ggtatacggc 540

cttccttcca gttacttgaa tttgaaataa aaaaaagttt gctgtcttgc tatcaagtat 600

aaatagacct gcaattatta atcttttgtt tcctcgtcat tgttctcgtt ccctttcttc 660

cttgtttctt tttctgcaca atatttcaag ctataccaag catacaatca actatctcat 720

atacaccatg g 731

<210> 6
<211> 496
<212> DNA
<213> Synechocystis sp. PCC6803

<400> 6
tcatgaagac cccattgacc gaagctgttt ctaccgcaga ttctcaaggt agattcttgt 60

catctaccga attgcaaatt gctttcggta gattgagaca agcaaatgct ggtttgcaag 120

ctgctaaggc tttgaccgat aacgctcaat ctttggttaa tggtgctgct caagctgttt 180

acaacaagtt cccatacacc actcaaaccc aaggtaacaa cttcgctgca gatcaaagag 240

gtaaggataa gtgtgctaga gatattggtt actacttgag aattgttacc tactgtttgg 300

ttgcaggtgg tactggtcca ttggatgaat acttgattgc tggtattgat gaaattaaca 360

gaaccttcga tttgtctcca tcttggtacg ttgaagcatt gaagtacatt aaggcaaatc 420

atggtttatc tggtgatgct agagatgaag caaactctta cttggattac gctattaacg 480

ctttgtctaa ctcgag 496

<210> 7
<211> 826
<212> DNA
<213> Synechocystis sp. PCC6803

<400> 7
tcatgagtga accaaacttg aacccagctt acaccttgga tcaagctatt gcaaacttgc 60

aacaaaccga agatgcttct gctagatact atgctgcttg gtggattggt agattcagag 120

ctgctcaacc agaaaccatt gctgctttgt tggttgcttt ggaagatgaa accgatagat 180

caccagatgg tggttaccca ttgagaagaa acgctgctaa ggctttgggt aaattgggtg 240

atagacaagt tgttccagct ttgattaagg ctttggaatg tgaagattac tacgttagag 300

aatctgctgc tcaagcattg gaaggtttgg gtgatgctag agctatggct ccattgatgg 360

ctaagttgac cggtggtttg gctgctgctc aattggttga aggtaagcca catttggctc 420

aaccatacga agctatcatt gaagcattgg gtactttgca agctgttgaa tctattggtt 480

tgattgaacc attcttggaa catttctcac caaaggttca atacgctgct gctagagctt 540

tgttccaatt gaccggtgat aacagatacg gtgatttgtt gattaccgct ttgggtggta 600

cagatttgca attgagaaga tcagctatga tggatttggg tgctactggt tacttaccag 660

gtgctcaagc tattgctaag gctttcgctg aaaactcttt gaagttgatt gctttgagag 720

atttgtgggc tacccataga caaagacaag catcttctga atctaaggct ttgtctccag 780

cttcaagaca aattttggaa ttgatggatt ctttgttgaa ctcgag 826

<210> 8
<211> 652
<212> DNA
<213> Synechocystis sp. PCC6803

<400> 8
ccatggaagg taactctgtt gttaccccag aaattgaaag attgattcaa gctgttgaaa 60

ccgcagattc tgctgctaag ttagttggtg ctgttagagc tttggctgct accagatcac 120

cattggctgt tccacaattg accaccgttt tgagatacaa caacccaggt gctgctgttg 180

ctgcagttga tggtttgatt caaattggtg atgctgctat gacccatttg ttggcaaaca 240

tggatggtta caactacggt gctagagctt gggctactag agcttgtgct ggtattggtg 300

atccaagagc tttggctttg ttgcaagaag ctgctttgac cgatttcgct ttgtctgtta 360

gaagagctgc tgctaagggt ttgggtttct tgagatggca atctttgcca caagaagaac 420

aagaaaccgt tcaaaaggct atttacgata ccttgattca agtttgtgaa gatccagaat 480

gggttgttag atacggtgct attgctggtt tggaaaactt ggctaagcaa gctcaacatt 540

acagacaacc attgaaggat ttcttgcaat ctttcgttga acaagaacca gaagctattg 600

ttggtgaaag aattttgtgg accttggaaa acattggtcc aattaactcg ag 652

<210> 9
<211> 730
<212> DNA
<213> Synechocystis sp. PCC6803

<400> 9
tcatgagtgt aaacttggct tcacaattga gagaaggtac taagaagtct cattctatgg 60

ctgaaaacgt tggtttcgtt aagtgtttct tgaagggtgt tgttgaaaag aactcttaca 120

gaaagttagt tggtaacttg tacttcgttt actctgctat ggaagaagaa atggctaagt 180

tcaaggatca tccaattttg tctcatatct acttcccaga attgaacaga aagcaatctt 240

tggaacaaga tttgcaattc tactacggtt caaactggag acaagaagtt aagatttctg 300

ctgctggtca agcatacgtt gatagagtta gacaagttgc tgctaccgct ccagaattgt 360

tggttgctca ttcttacacc agatacttgg gtgatttgtc tggtggtcaa attttgaaga 420

agattgctca aaacgctatg aacttgcatg atggtggtac tgctttctac gaatttgcag 480

atattgatga tgaaaaggct ttcaagaaca cctacagaca agctatgaac gatttgccaa 540

ttgatcaagc taccgctgaa agaattgttg atgaagcaaa cgatgctttc gctatgaaca 600

tgaagatgtt caacgaattg gaaggtaact tgattaaggc tattggtatt atggttttca 660

actctttgac cagaagaaga tcacaaggtt ctaccgaagt tggtttggct acctctgaag 720

gtaactcgag 730

<210> 10
<211> 754
<212> DNA
<213> Synechocystis sp. PCC6803

<400> 10
ccatggctgt taccgatttg tctttgacca actcttcttt gatgccaacc ttgaacccaa 60

tgattcaaca attggctttg gctattgctg cttcttggca atctttgcca ttgaagccat 120

accaattgcc agaagatttg ggttacgttg aaggcagatt ggaaggtgaa aagttggtta 180

ttgaaaacag atgttaccaa accccacaat tcagaaagat gcatttggaa ttggctaaag 240

ttggtaaggg tttggatatt ttgcattgtg ttatgttccc agaaccattg tacggtttgc 300

cattgttcgg ttgtgatatt gttgctggtc caggtggtgt ttctgctgct attgcagatt 360

tgtctccaac ccaatcagat agacaattgc cagctgctta ccaaaagtct ttggctgaat 420

tgggtcaacc agaatttgaa caacaaagag aattgccacc ttggggtgaa attttctctg 480

aatactgttt gttcattaga ccatcaaacg ttaccgaaga agaaagattc gttcaaagag 540

ttgttgattt cttgcaaatt cattgtcatc aatctattgt tgctgaacca ttgtctgaag 600

ctcaaacttt ggaacataga caaggtcaaa ttcattactg tcaacaacaa caaaagaacg 660

ataagaccag aagagttttg gaaaaggctt tcggtgaagc atgggctgaa agatacatgt 720

ctcaagtttt gttcgatgtt attcaaaact cgag 754

<210> 11
<211> 519
<212> DNA
<213> Synechocystis sp. PCC6803

<400> 11
atgttcgacg tattcactcg ggttgtttcc caagctgatg ctcgcggcga gtacctctct 60

ggttctcagt tagatgcttt gagcgctacc gttgctgaag gcaacaaacg gattgattct 120

gttaaccgca tcaccggtaa tgcttccgct atcgtttcca acgctgctcg tgctttgttc 180

gttgaacagc cccaattaat ccaacccggt ggaaacgcct acaccagccg tcgtatggct 240

gcttgtttgc gtgacatgga aatcatcctc cgctatgtta cctacgcaac cttcaccggc     300

gacgcttccg ttctagaaga tcgttgcttg aacggtctcc gtgaaaccta cgttgccctg     360

ggtgttcccg gtgcttccgt agctgctggc gttcaaaaaa tgaaagaagc tgccctggac     420

atcgttaacg atcccaatgg catcacccgt ggtgattgca gtgctatcgt tgctgaaatc     480

gctggttact tcgaccgcgc cgctgctgcc gtagcctag                            519

<210>  12
<211>  172
<212>  PRT
<213>  synthetisch

<400>  12

Met Phe Asp Val Phe Thr Arg Val Val Ser Gln Ala Asp Ala Arg Gly
1               5                   10                  15

Glu Tyr Leu Ser Gly Ser Gln Leu Asp Ala Leu Ser Ala Thr Val Ala
            20                  25                  30

Glu Gly Asn Lys Arg Ile Asp Ser Val Asn Arg Ile Thr Gly Asn Ala
        35                  40                  45

Ser Ala Ile Val Ser Asn Ala Ala Arg Ala Leu Phe Val Glu Gln Pro
    50                  55                  60

Gln Leu Ile Gln Pro Gly Gly Asn Ala Tyr Thr Ser Arg Arg Met Ala
65                  70                  75                  80

Ala Cys Leu Arg Asp Met Glu Ile Ile Leu Arg Tyr Val Thr Tyr Ala
                85                  90                  95

Thr Phe Thr Gly Asp Ala Ser Val Leu Glu Asp Arg Cys Leu Asn Gly
            100                 105                 110

Leu Arg Glu Thr Tyr Val Ala Leu Gly Val Pro Gly Ala Ser Val Ala
        115                 120                 125

Ala Gly Val Gln Lys Met Lys Glu Ala Ala Leu Asp Ile Val Asn Asp
    130                 135                 140

Pro Asn Gly Ile Thr Arg Gly Asp Cys Ser Ala Ile Val Ala Glu Ile
145                 150                 155                 160

Ala Gly Tyr Phe Asp Arg Ala Ala Ala Ala Val Ala
                165                 170

<210>  13
<211>  10
<212>  DNA
<213>  synthetisch

<400> 13
actctgtaga 10

<210> 14
<211> 11874
<212> DNA
<213> synthetisch

<400> 14
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accacgcttt tcaattcaat tcatcatttt ttttttattc ttttttttga tttcggtttc 240
tttgaaattt ttttgattcg gtaatctccg aacagaagga agaacgaagg aaggagcaca 300
gacttagatt ggtatatata cgcatatgta gtgttgaaga aacatgaaat tgcccagtat 360
tcttaaccca actgcacaga acaaaaacct gcaggaaacg aagataaatc atgtcgaaag 420
ctacatataa ggaacgtgct gctactcatc ctagtcctgt tgctgccaag ctatttaata 480
tcatgcacga aaagcaaaca aacttgtgtg cttcattgga tgttcgtacc accaaggaat 540
tactggagtt agttgaagca ttaggtccca aaatttgttt actaaaaaca catgtggata 600
tcttgactga tttttccatg gagggcacag ttaagccgct aaaggcatta tccgccaagt 660
acaatttttt actcttcgaa gacagaaaat ttgctgacat tggtaataca gtcaaattgc 720
agtactctgc gggtgtatac agaatagcag aatgggcaga cattacgaat gcacacggtg 780
tggtgggccc aggtattgtt agcggtttga agcaggcggc agaagaagta acaaaggaac 840
ctagaggcct tttgatgtta gcagaattgt catgcaaggg ctccctatct actggagaat 900
atactaaggg tactgttgac attgcgaaga gcgacaaaga ttttgttatc ggctttattg 960
ctcaaagaga catgggtgga agagatgaag gttacgattg gttgattatg acacccggtg 1020
tgggtttaga tgacaaggga gacgcattgg gtcaacagta tagaaccgtg gatgatgtgg 1080
tctctacagg atctgacatt attattgttg gaagaggact atttgcaaag ggaagggatg 1140
ctaaggtaga gggtgaacgt tacagaaaag caggctggga agcatatttg agaagatgcg 1200
gccagcaaaa ctaaaaaact gtattataag taaatgcatg tatactaaac tcacaaatta 1260
gagcttcaat ttaattatat cagttattac cctgcggtgt gaaataccgc acagatgcgt 1320
aaggagaaaa taccgcatca ggaaattgta aacgttaata ttttgttaaa attcgcgtta 1380
aatttttgtt aaatcagctc attttttaac caataggccg aaatcggcaa aatcccttat 1440
aaatcaaaag aatagaccga gatagggttg agtgttgttc cagtttggaa caagagtcca 1500
ctattaaaga acgtggactc caacgtcaaa gggcgaaaaa ccgtctatca gggcgatggc 1560
ccactacgtg aaccatcacc ctaatcaagt tttttggggt cgaggtgccg taaagcacta 1620
aatcggaacc ctaaagggag cccccgattt agagcttgac ggggaaagcc ggcgaacgtg 1680
gcgagaaagg aagggaagaa agcgaaagga gcgggcgcta gggcgctggc aagtgtagcg 1740

gtcacgctgc gcgtaaccac cacacccgcc gcgcttaatg cgccgctaca gggcgcgtcg 1800
cgccattcgc cattcaggct gcgcaactgt tgggaagggc gatcggtgcg ggcctcttcg 1860
ctattacgcc agctggcgaa ggggggatgt gctgcaaggc gattaagttg ggtaacgcca 1920
gggttttccc agtcacgacg ttgtaaaacg acggccagtg aattgtaata cgactcacta 1980
tagggcgaat tggagctcat atccttttgt tgtttccggg tgtacaatat ggacttcctc 2040
ttttctggca accaaaccca tacatcggga ttcctataat accttcgttg gtctccctaa 2100
catgtaggtg gcggagggga gatatacaat agaacagata ccagacaaga cataatgggc 2160
taaacaagac tacaccaatt acactgcctc attgatggtg gtacataacg aactaatact 2220
gtagccctag acttgatagc catcatcata tcgaagtttc actacccttt ttccatttgc 2280
catctattga agtaataata ggcgcatgca acttcttttc tttttttttc ttttctctct 2340
cccccgttgt tgtctcacca tatccgcaat gacaaaaaaa tgatggaaga cactaaagga 2400
aaaaattaac gacaaagaca gcaccaacag atgtcgttgt tccagagctg atgaggggta 2460
tctcgaagca cacgaaactt tttccttcct tcattcacgc acactactct ctaatgagca 2520
acggtatacg gccttccttc cagttacttg aatttgaaat aaaaaaaagt ttgctgtctt 2580
gctatcaagt ataaatagac ctgcaattat taatcttttg tttcctcgtc attgttctcg 2640
ttccctttct tccttgtttc tttttctgca caatatttca agctatacca agcatacaat 2700
caactatctc atatacacca tggaaggtaa ctctgttgtt accccagaaa ttgaaagatt 2760
gattcaagct gttgaaaccg cagattctgc tgctaagtta gttggtgctg ttagagcttt 2820
ggctgctacc agatcaccat tggctgttcc acaattgacc accgttttga gatacaacaa 2880
cccaggtgct gctgttgctg cagttgatgg tttgattcaa attggtgatg ctgctatgac 2940
ccatttgttg gcaaacatgg atggttacaa ctacggtgct agagcttggg ctactagagc 3000
ttgtgctggt attggtgatc caagagcttt ggctttgttg caagaagctg ctttgaccga 3060
tttcgctttg tctgttagaa gagctgctgc taagggtttg ggtttcttga gatggcaatc 3120
tttgccacaa gaagaacaag aaaccgttca aaaggctatt tacgatacct tgattcaagt 3180
ttgtgaagat ccagaatggg ttgttagata cggtgctatt gctggtttgg aaaacttggc 3240
taagcaagct caacattaca gacaaccatt gaaggatttc ttgcaatctt tcgttgaaca 3300
agaaccagaa gctattgttg gtgaaagaat tttgtggacc ttggaaaaca ttggtccaat 3360
taactcgaga taaggtatat aactctgtag aaataaagag tatcatcttt caaaccgcgg 3420
atatcctttt gttgtttccg ggtgtacaat atggacttcc tcttttctgg caaccaaacc 3480
catacatcgg gattcctata ataccttcgt tggtctccct aacatgtagg tggcggaggg 3540
gagatataca atagaacaga taccagacaa gacataatgg gctaaacaag actacaccaa 3600
ttacactgcc tcattgatgg tggtacataa cgaactaata ctgtagccct agacttgata 3660
gccatcatca tatcgaagtt tcactaccct ttttccattt gccatctatt gaagtaataa 3720
taggcgcatg caacttcttt tctttttttt tcttttctct ctcccccgtt gttgtctcac 3780

catatccgca atgacaaaaa aatgatggaa gacactaaag gaaaaaatta acgacaaaga 3840
cagcaccaac agatgtcgtt gttccagagc tgatgagggg tatctcgaag cacacgaaac 3900
tttttccttc cttcattcac gcacactact ctctaatgag caacggtata cggccttcct 3960
tccagttact tgaatttgaa ataaaaaaaa gtttgctgtc ttgctatcaa gtataaatag 4020
acctgcaatt attaatcttt tgtttcctcg tcattgttct cgttcccttt cttccttgtt 4080
tctttttctg cacaatattt caagctatac caagcataca atcaactatc tcatatacac 4140
catggctgtt accgatttgt ctttgaccaa ctcttctttg atgccaacct tgaacccaat 4200
gattcaacaa ttggctttgg ctattgctgc ttcttggcaa tctttgccat tgaagccata 4260
ccaattgcca gaagatttgg gttacgttga aggcagattg gaaggtgaaa agttggttat 4320
tgaaaacaga tgttaccaaa ccccacaatt cagaaagatg catttggaat tggctaaagt 4380
tggtaagggt ttggatattt tgcattgtgt tatgttccca gaaccattgt acggtttgcc 4440
attgttcggt tgtgatattg ttgctggtcc aggtggtgtt tctgctgcta ttgcagattt 4500
gtctccaacc caatcagata gacaattgcc agctgcttac caaaagtctt tggctgaatt 4560
gggtcaacca gaatttgaac aacaaagaga attgccacct tggggtgaaa ttttctctga 4620
atactgtttg ttcattagac catcaaacgt taccgaagaa gaaagattcg ttcaaagagt 4680
tgttgatttc ttgcaaattc attgtcatca atctattgtt gctgaaccat tgtctgaagc 4740
tcaaactttg gaacatagac aaggtcaaat tcattactgt caacaacaac aaaagaacga 4800
taagaccaga agagttttgg aaaaggcttt cggtgaagca tgggctgaaa gatacatgtc 4860
tcaagttttg ttcgatgtta ttcaaaactc gagataaggt atataactct gtagaaataa 4920
agagtatcat ctttcaaacc gcggattgtc gcgatcaaat cgatatgtct tatgcggccg 4980
catatccttt tgttgtttcc gggtgtacaa tatggacttc ctcttttctg gcaaccaaac 5040
ccatacatcg ggattcctat aataccttcg ttggtctccc taacatgtag gtggcggagg 5100
ggagatatac aatagaacag ataccagaca agacataatg ggctaaacaa gactacacca 5160
attacactgc ctcattgatg gtggtacata acgaactaat actgtagccc tagacttgat 5220
agccatcatc atatcgaagt ttcactaccc tttttccatt tgccatctat tgaagtaata 5280
ataggcgcat gcaacttctt ttcttttttt ttcttttctc tctcccccgt tgttgtctca 5340
ccatatccgc aatgacaaaa aaatgatgga agacactaaa ggaaaaaatt aacgacaaag 5400
acagcaccaa cagatgtcgt tgttccagag ctgatgaggg gtatctcgaa gcacacgaaa 5460
ctttttcctt ccttcattca cgcacactac tctctaatga gcaacggtat acggccttcc 5520
ttccagttac ttgaatttga aataaaaaaa agtttgctgt cttgctatca agtataaata 5580
gacctgcaat tattaatctt ttgtttcctc gtcattgttc tcgttccctt tcttccttgt 5640
ttctttttct gcacaatatt tcaagctata ccaagcatac aatcaactat ctcatataca 5700
ccatgagtga accaaacttg aacccagctt acaccttgga tcaagctatt gcaaacttgc 5760
aacaaaccga agatgcttct gctagatact atgctgcttg gtggattggt agattcagag 5820

ctgctcaacc agaaaccatt gctgctttgt tggttgcttt ggaagatgaa accgatagat 5880
caccagatgg tggttaccca ttgagaagaa acgctgctaa ggctttgggt aaattgggtg 5940
atagacaagt tgttccagct ttgattaagg ctttggaatg tgaagattac tacgttagag 6000
aatctgctgc tcaagcattg gaaggtttgg gtgatgctag agctatggct ccattgatgg 6060
ctaagttgac cggtggtttg gctgctgctc aattggttga aggtaagcca catttggctc 6120
aaccatacga agctatcatt gaagcattgg gtactttgca agctgttgaa tctattggtt 6180
tgattgaacc attcttggaa catttctcac caaaggttca atacgctgct gctagagctt 6240
tgttccaatt gaccggtgat aacagatacg gtgatttgtt gattaccgct ttgggtggta 6300
cagatttgca attgagaaga tcagctatga tggatttggg tgctactggt tacttaccag 6360
gtgctcaagc tattgctaag gctttcgctg aaaactcttt gaagttgatt gctttgagag 6420
atttgtgggc tacccataga caaagacaag catcttctga atctaaggct ttgtctccag 6480
cttcaagaca aattttggaa ttgatggatt ctttgttgaa ctcgagataa ggtatataac 6540
tctgtagaaa taaagagtat catctttcaa accgcggatt gtcgcgatca aatcgatatg 6600
tcttatgcgg ccgcttacga ccgttaactt gttctagaat atccttttgt tgtttccggg 6660
tgtacaatat ggacttcctc ttttctggca accaaaccca tacatcggga ttcctataat 6720
accttcgttg gtctccctaa catgtaggtg gcggagggga gatatacaat agaacagata 6780
ccagacaaga cataatgggc taaacaagac tacaccaatt acactgcctc attgatggtg 6840
gtacataacg aactaatact gtagccctag acttgatagc catcatcata tcgaagtttc 6900
actacccttt ttccatttgc catctattga agtaataata ggcgcatgca acttcttttc 6960
tttttttttc ttttctctct cccccgttgt tgtctcacca tatccgcaat gacaaaaaaa 7020
tgatggaaga cactaaagga aaaaattaac gacaaagaca gcaccaacag atgtcgttgt 7080
tccagagctg atgaggggta tctcgaagca cacgaaactt tttccttcct tcattcacgc 7140
acactactct ctaatgagca acggtatacg gccttccttc cagttacttg aatttgaaat 7200
aaaaaaaagt ttgctgtctt gctatcaagt ataaatagac ctgcaattat taatcttttg 7260
tttcctcgtc attgttctcg ttccctttct tccttgtttc tttttctgca caatatttca 7320
agctatacca agcatacaat caactatctc atatacacca tgagtgtaaa cttggcttca 7380
caattgagag aaggtactaa gaagtctcat tctatggctg aaaacgttgg tttcgttaag 7440
tgtttcttga agggtgttgt tgaaaagaac tcttacagaa agttagttgg taacttgtac 7500
ttcgtttact ctgctatgga agaagaaatg gctaagttca aggatcatcc aattttgtct 7560
catatctact tcccagaatt gaacagaaag caatctttgg aacaagattt gcaattctac 7620
tacggttcaa actggagaca agaagttaag atttctgctg ctggtcaagc atacgttgat 7680
agagttagac aagttgctgc taccgctcca gaattgttgg ttgctcattc ttacaccaga 7740
tacttgggtg atttgtctgg tggtcaaatt ttgaagaaga ttgctcaaaa cgctatgaac 7800
ttgcatgatg gtggtactgc tttctacgaa tttgcagata ttgatgatga aaaggctttc 7860

aagaacacct acagacaagc tatgaacgat ttgccaattg atcaagctac cgctgaaaga 7920
attgttgatg aagcaaacga tgctttcgct atgaacatga agatgttcaa cgaattggaa 7980
ggtaacttga ttaaggctat tggtattatg gttttcaact ctttgaccag aagaagatca 8040
caaggttcta ccgaagttgg tttggctacc tctgaaggta actcgagata aggtatataa 8100
ctctgtagaa ataaagagta tcatctttca aaccgcggat tgtcgcgatc aaatcgatat 8160
gtcttatgcg gccgcttacg accgttaact tgttctagat tgggctagcg ttgagatcta 8220
tatccttttg ttgtttccgg gtgtacaata tggacttcct cttttctggc aaccaaaccc 8280
atacatcggg attcctataa taccttcgtt ggtctcccta acatgtaggt ggcggagggg 8340
agatatacaa tagaacagat accagacaag acataatggg ctaaacaaga ctacaccaat 8400
tacactgcct cattgatggt ggtacataac gaactaatac tgtagcccta gacttgatag 8460
ccatcatcat atcgaagttt cactaccctt tttccatttg ccatctattg aagtaataat 8520
aggcgcatgc aacttctttt cttttttttt cttttctctc tcccccgttg ttgtctcacc 8580
atatccgcaa tgacaaaaaa atgatggaag acactaaagg aaaaaattaa cgacaaagac 8640
agcaccaaca gatgtcgttg ttccagagct gatgaggggt atctcgaagc acacgaaact 8700
ttttccttcc ttcattcacg cacactactc tctaatgagc aacggtatac ggccttcctt 8760
ccagttactt gaatttgaaa taaaaaaaag tttgctgtct tgctatcaag tataaataga 8820
cctgcaatta ttaatctttt gtttcctcgt cattgttctc gttccctttc ttccttgttt 8880
ctttttctgc acaatatttc aagctatacc aagcatacaa tcaactatct catatacacc 8940
atgaagaccc cattgaccga agctgtttct accgcagatt ctcaaggtag attcttgtca 9000
tctaccgaat tgcaaattgc tttcggtaga ttgagacaag caaatgctgg tttgcaagct 9060
gctaaggctt tgaccgataa cgctcaatct ttggttaatg gtgctgctca agctgtttac 9120
aacaagttcc catacaccac tcaaacccaa ggtaacaact tcgctgcaga tcaaagaggt 9180
aaggataagt gtgctagaga tattggttac tacttgagaa ttgttaccta ctgtttggtt 9240
gcaggtggta ctggtccatt ggatgaatac ttgattgctg gtattgatga aattaacaga 9300
accttcgatt tgtctccatc ttggtacgtt gaagcattga agtacattaa ggcaaatcat 9360
ggtttatctg gtgatgctag agatgaagca aactcttact tggattacgc tattaacgct 9420
ttgtctaact cgagataagg tatataactc tgtagaaata aagagtatca tctttcaaac 9480
cgcggattgt cgcgatcaaa tcgatatgtc ttatgcggcc gcttacgacc gttaacttgt 9540
tctagattgg gctagcgttg agatctttag aaacgtcgac ctcgaggggg ggcccggtac 9600
ccagcttttg ttccctttag tgagggttaa ttccgagctt ggcgtaatca tggtcatagc 9660
tgtttcctgt gtgaaattgt tatccgctca caattccaca caacatagga gccggaagca 9720
taaagtgtaa agcctggggt gcctaatgag tgaggtaact cacattaatt gcgttgcgct 9780
cactgcccgc tttccagtcg ggaaacctgt cgtgccagct gcattaatga atcggccaac 9840
gcgcggggag aggcggtttg cgtattgggc gctcttccgc ttcctcgctc actgactcgc 9900

tgcgctcggt cgttcggctg cggcgagcgg tatcagctca ctcaaaggcg gtaatacggt 9960
tatccacaga atcaggggat aacgcaggaa agaacatgtg agcaaaaggc cagcaaaagg 10020
ccaggaaccg taaaaaggcc gcgttgctgg cgtttttcca taggctcggc ccccctgacg 10080
agcatcacaa aaatcgacgc tcaagtcaga ggtggcgaaa cccgacagga ctataaagat 10140
accaggcgtt tccccctgga agctccctcg tgcgctctcc tgttccgacc ctgccgctta 10200
ccggatacct gtccgccttt ctcccttcgg gaagcgtggc gctttctcaa tgctcacgct 10260
gtaggtatct cagttcggtg taggtcgttc gctccaagct gggctgtgtg cacgaacccc 10320
ccgttcagcc cgaccgctgc gccttatccg gtaactatcg tcttgagtcc aacccggtaa 10380
gacacgactt atcgccactg gcagcagcca ctggtaacag gattagcaga gcgaggtatg 10440
taggcggtgc tacagagttc ttgaagtggt ggcctaacta cggctacact agaaggacag 10500
tatttggtat ctgcgctctg ctgaagccag ttaccttcgg aaaaagagtt ggtagctctt 10560
gatccggcaa acaaaccacc gctggtagcg gtggtttttt tgtttgcaag cagcagatta 10620
cgcgcagaaa aaaaggatct caagaagatc ctttgatctt ttctacgggg tctgacgctc 10680
agtggaacga aaactcacgt taagggattt tggtcatgag attatcaaaa aggatcttca 10740
cctagatcct tttaaattaa aaatgaagtt ttaaatcaat ctaaagtata tatgagtaaa 10800
cttggtctga cagttaccaa tgcttaatca gtgaggcacc tatctcagcg atctgtctat 10860
ttcgttcatc catagttgcc tgactgcccg tcgtgtagat aactacgata cgggagggct 10920
taccatctgg ccccagtgct gcaatgatac cgcgagaccc acgctcaccg gctccagatt 10980
tatcagcaat aaaccagcca gccggaaggg ccgagcgcag aagtggtcct gcaactttat 11040
ccgcctccat ccagtctatt aattgttgcc gggaagctag agtaagtagt tcgccagtta 11100
atagtttgcg caacgttgtt gccattgcta caggcatcgt ggtgtcacgc tcgtcgtttg 11160
gtatggcttc attcagctcc ggttcccaac gatcaaggcg agttacatga tcccccatgt 11220
tgtgaaaaaa agcggttagc tccttcggtc ctccgatcgt tgtcagaagt aagttggccg 11280
cagtgttatc actcatggtt atggcagcac tgcataattc tcttactgtc atgccatccg 11340
taagatgctt ttctgtgact ggtgagtact caaccaagtc attctgagaa tagtgtatgc 11400
ggcgaccgag ttgctcttgc ccggcgtcaa tacgggataa taccgcgcca catagcagaa 11460
ctttaaaagt gctcatcatt ggaaaacgtt cttcggggcg aaaactctca aggatcttac 11520
cgctgttgag atccagttcg atgtaaccca ctcgtgcacc caactgatct tcagcatctt 11580
ttactttcac cagcgtttct gggtgagcaa aaacaggaag gcaaaatgcc gcaaaaaagg 11640
gaataagggc gacacggaaa tgttgaatac tcatactctt cctttttcaa tattattgaa 11700
gcatttatca gggttattgt ctcatgagcg gatacatatt tgaatgtatt tagaaaaata 11760
aacaaatagg ggttccgcgc acatttcccc gaaaagtgcc acctgacgtc taagaaacca 11820
ttattatcat gacattaacc tataaaaata ggcgtatcac gaggcccttt cgtc 11874

## Beschreibung

Die Erfindung betrifft rekombinant hergestelltes Phycocyanin bzw. funktionell aktive Teile davon.

In allen Bereichen der modernen Biologie - z.B. bei der DNA-Replikation, Transkription, Translation oder Kontrolle des Zellzyklus sowie Signaltransduktionen spielt die Wechselwirkung zwischen Proteinen eine essentielle Rolle. Konsequenterweise ist die Aufklärung dieser Abläufe nicht mehr nur allein die Domäne von Biochemikem. Vielmehr müssen sich nun auch Genetiker sowie Molekularbiologen oder Biophysiker mit der Aufklärung von Protein-Protein-Wechselwirkungen beschäftigen.

Die Interaktionen zwischen Proteinen werden klassischerweise mit Hilfe biochemischer Methoden wie der Affinitätschromatographie oder der Immunpräzipitation identifiziert oder quantifiziert. Häufig werden diese Methoden mit genetischen bzw. molekularbiologischen Verfahren kombiniert. Wird beispielsweise ein gesuchtes Protein von einer Bakteriophagen/cDNA Bibliothek exprimiert, kann die Interaktion in manchen Fällen mit einer spezifischen Protein-Sonde direkt im Lysat eines positiven Plaques nachgewiesen werden.

Eine Vielzahl der Verfahren zur Untersuchung von Protein-Protein-Wechselwirkungen beruht jedoch auf der Detektion dieser Wechselwirkungen *in vitro.* So ist zum einen die Übertragbarkeit der damit erzeugten Ergebnisse auf *in vivo* Bedingungen fraglich. Des weiteren können nur Wechselwirkungen detektiert werden von Bindungspartnem, deren Dissoziationskonstanten nicht allzu groß sind. Anderenfalls würden die zu detektierenden Wechselwirkungen den in der Regel stringenten und zeitaufwendigen Waschprotokollen nicht standhalten. Diese sind aber üblicherweise notwendig, um die störenden Hintergrundsignale auf ein akzeptables Maß senken zu können. Die Entwicklung von Systemen zur Untersuchung der Protein-Protein-Wechselwirkungen *in vivo* stellt daher eine besondere Herausforderung dar.

In der jüngeren Vergangenheit verwenden viele Wissenschaftler zur Untersuchung der Zellbiologie das sogenannte grüne Fluoreszenzprotein (GFP) aus *Aequorea victoria.* Bei GFP handelt es sich um ein fluoreszierendes Protein, dessen Klonierung und Expression in der Zwischenzeit in einer Vielzahl von Zellen und Organismen - z. B. in Bakterien, Pflanzen- und Tierzellen - gelungen ist. Damit kann das GFP in der Zellbiologie beispielsweise für die Lokalisation von Proteinen, für den Nachweis einer erfolgreichen Transfektion oder als Reportergen für eine Genexpression dienen.

Das GFP zeigt ein Absorptionsspektrum mit zwei Peaks, nämlich einen ersten Peak bei 395 nm und einen zweiten bei 470 nm. Dafür ist das Tyr⁶⁶ verantwortlich, das in seiner Hydroxy-Form durch die Anregung bei 395 nm in die Phenolat-Form übergeht. Diese emittiert nach entsprechender Anregung Licht einer Wellenlänge von 509 nm.

Damit liegt das Emissions- und Absorptionsspektrum des GFP genau in den spektralen Bereichen, in denen eine Vielzahl biologischer Substanzen eine Fluoreszenz aufweisen. Diese natürliche Fluoreszenz biologischer Systeme - auch als Autofluoreszenz bekannt - geht zu nicht unerheblichen Teilen auf Flavinderivate oder auf NADH- sowie NADPH-Derivate zurück. So werden Flavine beispielsweise durch Licht von 380-490 nm angeregt und emittieren Licht einer Wellenlänge von 520-560 nm. NADH sowie NADPH zeigen Absorptionsmaxima bei 360-390 nm, während sie die höchste Emission bei 440-470 nm aufweisen.

Diese Autofluoreszenz überschneidet sich daher mit dem für das GFP Protein typischen Spektrum, und ist bei der Verwendung von GFP in *in vivo* Studien für ein hohes Hintergrundrauschen verantwortlich. Dieses Hintergrundrauschen führt nicht selten zu einem Verlust an Klarheit und Aussagekraft der Versuchsergebnisse.

In der Vergangenheit wurden Verfahren entwickelt, um die Autofluoreszenz von der GFP Fluoreszenz zu unterscheiden. Diese Verfahren beruhen beispielsweise auf der Optimierung der für die Fluoreszenzdetektion verwendeten optischen Filter oder auf der sequentiellen Anregung der Probe zunächst mit einer auf die Anregung des GFP optimierten Wellenlänge und im Anschluß daran mit einer auf die Autofluoreszenz optimierten Wellenlänge. Die Differenz zwischen den erhabenen Meßsignalen gilt als Fluoreszenz des GFP allein. Diese letzte Methode ist als "Dual Wavelength Correction" bekannt. Auch andere Verfahren, so beispielsweise die "Microscopy Image Correction" basiert letztlich auf einer Korrektur des GFP Fluoreszenzsignals um den Wert der Autofluoreszenz.

Daneben wurden aufwendige Optimierungen an der eigentlichen Auswerteeinheit vorgenommen. So wird beispielsweise die konfokale Lasermikroskopie oder die Zwei- Photonen-Laserscan-Mikroskopie eingesetzt, um eine hohe Auflösung der Fluoreszenzsignale zu erhalten und sie somit möglichst einzelnen konkreten dreidimensionalen oder räumlichen Strukturen innerhalb der biologischen Probe zuordnen zu können. Dieser letzte Ansatz geht auf Beobachtungen zurück, dass die Autofluoreszenz innerhalb der Probe meist nicht homogen verteilt ist.

Ein Großteil der Überlegungen zur Verbesserung der Auswertung eines GFP-Signals in einer *in vivo* Probe setzt daher an der Gestaltung des Messverfahrens sowie an einer Optimierung der Detektions- oder Auswerteeinheit an. (siehe dazu ausführlich: Billinton N und A.W. Knight: Seeing the Wood through the Trees: A Review of Techniques for distinguishing GFP from Cellular endogenous Autofluorescence. Analytical Biochemistry 2001 (291 ), 175-197).

Daneben existieren nur wenige Ansätze, das GFP durch Mutagenese so zu verändern, dass die Absorption und die Emission ins längerwellige Licht verschoben wird und somit den kritischen Bereich der Autofluoreszenz verlässt. So wurde z. B. das sogenannten Red Fluorescent Protein (RFP oder DsRed) aus *Discosoma* mit einem Emissionsmaximum bei 583 nm isoliert, dessen Weiterentwicklung mRFP1 ein Absorptionsmaximum bei 619 nm zeigt. Diese Variante hat zwar - im Gegensatz zum ursprünglichen RFP - keinen störenden zweiten Peak bei 490 nm. Es ist aber durch eine demgegenüber geringere Photostabilität und Signalstärke gekennzeichnet (Zhang J., E. Campbell, A. Ting und Y. Tsien: Creating New Fluorescent Probes for Cell Biology. Molecular Cell Biology 2002 (3), S. 906-918).

Aufgabe der vorliegenden Erfindung ist es daher, ein alternatives Fluoreszenzprotein insbesondere für zellbiologische Untersuchungen, sowie ein Verfahren zu seiner Herstellung bereitzustellen. Dieses Protein sollte insbesondere ein Absorptionsmaximum im längerwelligen Bereich aufweisen.

Gelöst wird diese Aufgabe durch die Bereitstellung eines Phycocyanins oder funktionell aktiver Teile davon, wobei das Phycocyanin bzw. dessen erfindungsgemäße Teile im Vergleich zum nativen Phycocyanin eine Glycosylierung aufweisen. Des weiteren wird eine transformierte Eukaryontenzelle, insbesondere eine Hefezelle, bereitgestellt, die das erfindungsgemäße Phycocyanin bzw. dessen funktionell aktive Teile exprimiert.

Bei Phycocyaninen handelt es sich um Phycobiliproteine, die als akzessorische Photosynthesepigmente aus Cyanobakterien, Rotalgen oder Cryptomonaden bekannt sind. In Cyanobakterien und Rotalgen liegen einzelne Phycobiliproteine überwiegend aggregiert zu großen Untereinheiten, den sogenannten Phycobilisomen, vor.

Die Phycobiliproteine gehören zu der Familie homologer Proteine, die jeweils aus einem eng miteinander verbundenen αβ-Heterodimer bestehen. Beide Untereinheiten - also sowohl die α- als auch die β-Untereinheit können ein oder mehrere Chromophoren enthalten. Die Qualität und Anzahl der kovalent an die Proteineinheit (Apo-Protein) gebunden Chromophore dient der Klassifizierung der Phycobiliproteine.

Die Phycocyanine tragen eine einzige Bilin-Einheit an der α-Untereinheit und zwei Biline an der β-Untereinheit. Im Vergleich dazu sind beispielsweise Phycoerythrine durch zwei oder drei Biline an der α-Untereinheit und drei an der β-Untereinheit charakterisiert. Allophycocyanine hingegen tragen an jeder Proteinuntereinheit nur jeweils ein Bilin.

Wie bereits erwähnt, werden die Biline aufgrund ihrer Struktur unterschieden. Bei allen Bilinen handelt es sich jedoch übereinstimmend um Chromophore aus einem linearen Tetrapyrrol, das über eine Thioetherbindung zwischen dem C3'-Atom des Bilins und einem Cysteinrest der Proteineinheit kovalent an das Apo-Protein gebunden ist. Die Anzahl der Thioetherbindungen kann jedoch variieren. So weisen einige Biline zwei Thioetherbindungen zum Apo-Protein auf. Ebenso kann die Konfiguration des C3'-Atoms R oder S sein.

Die spektroskopischen Eigenschaften der Phycobiliproteine werden wesentlich durch die Anzahl und die Lage der Doppelbindungen in dem Bilin bestimmt. So wird zwischen dem Phycocyanobilin (PCB), dem Phycobiliviolin (PXB), dem Phycoerythrobilin (PEB) und dem Phycourobilin (PUB) unterschieden. Die zwei Thioetherbindungen zum Apo-Protein aufweisenden PEB und PUB tragen die Bezeichnung DiCysPEB bzw. DiCysPUB.

Daher ist die Konformation des Bilin-Anteils für die spektralen Eigenschaften des Phycobiliproteins von großer Bedeutung. So zeigen Röntgenstrukturanalysen, dass das Bilin im Phycobiliprotein in der Regel eine möglichst gestreckte Konformation einnimmt. Liegt das Bilin dagegen frei in Lösung vor - wodurch es eine davon abweichende Konformation einnehmen kann - verschiebt sich das Absorptionsmaximum stärker in den UV-Bereich.

Das Phycocyanin ist - wie bereits erwähnt - durch ein Bilin an der α-Untereinheit und zwei Biline an der β-Untereinheit gekennzeichnet. Bei dem Bilin der α-Untereinheit handelt es sich stets um ein PCB. Die β-Untereinheit kann entweder zwei PCB oder aber ein PCB und PEB tragen. Im ersten Fall handelt es sich um das C-Phycocyanin (C-PC), während das zweite Molekül als R-Phycocyanin (R-PC) bezeichnet wird. Die beiden PC-Varianten unterscheiden sich allerdings in ihren spektralen Eigenschaften nicht wesentlich.

Die meisten Phycobiliproteine absorbieren im Bereich von 495-650 nm. Phycocyanin zeigt - je nach Literatur, Versuchsbedingungen und Ausgangsorganismus - ein Absorptionsmaximum bei 600-650 nm. Nach Tooley *et al* (Tooley A.J., Yuping A.C., Glazer A.N.: Biosysnthesis of a fluorescent cyanobacterial C-Phycocyanin holo-α, subunit in a heterologous host. PNAS 2001 Vol 19) 10560 - 10565) zeigt das Holo-CpcA (d.h. also die α-Untereinheit des PC) ein Absorptionsmaximum von 619 nm und eine maximale Emission bei 641 nm.

Diese spektralen Eigenschaften des Phycocyanins erlauben eine klare Diskriminierung des eigentlichen Fluoreszenz-Meßsignals von dem störenden Hintergrundrauschen der Autofluoreszenz und begründen somit dessen besondere Eignung als Fluoreszenzmarker für in vivo-Untersuchungen. Die Autofluoreszenz beruht - wie bereits oben erwähnt - im wesentlichen auf Flavin- oder NADH- bzw. NADPH-Derivaten, die Absorption- und Emissionsmaxima bei 360-560 nm zeigen.

Die grundlegenden Untersuchungen zur Biosynthese des Phycocyanins wurden an der Rotalge *Cyanidium caldarium* gemacht. Dabei wurde durch ¹⁴C-Markierungen des Häms deutlich, dass die Biosynthese des Phycocyanins zunächst die Degradation des endogenen Häms zu Biliverdin IXα durch die Hämoxygenase 1 (HO1) voraussetzt. Dabei wird der Tetrapyrrolring linearisiert. In weiteren Schritten wird das Biliverdin zu 14,16 Dihydrobiliverdin IXα und anschließend zu 3 Z - Phycocyanobilin umgewandelt.

Diese letzten beiden Umwandlungen setzen jeweils die Gegenwart von reduziertem Ferredoxin voraus und werden durch die Ferredoxin Oxidoreduktase katalysiert. Das so entstandene PCB wird durch die heterodimere Phycocyanin α PCB Lyase an die α-Untereinheit des Phycobiliproteins (Apo-CpcA) geknüpft. Damit entsteht die fertige Holo-α-Untereinheit (Holo-CpcA)

Die Biosynthese des Holo-CpcA umfaßt somit zunächst die Degradation und Umwandlung des Häms zum PCB und anschließend dessen enzymatische Bindung an die Proteinuntereinheit. Entsprechend verläuft die Biosynthese der Holo-β-Untereinheit (Holo-CpcB), wobei die Bindung der Bilin-Einheiten an das CpcB vermutlich zumindest teilweise nicht-enzymatisch erfolgt. Liegen sowohl das Holo-CpcA als auch das Holo-CpcB vor, dimerisieren diese zu einem "Monomer" des Phycocyanins.

Die vollständige Biosynthese des Holo-CpcA wurde erstmals in der photosynthetisierenden Blaualge *Synechocystis sp.* PCC 6803 erkannt und die dafür relevanten Gene identifiziert (Nakamura, Y., Kaneko T., Hirosawa M., Miyajima N., Tabata S. (1998) Nucleic Acids Res. 26, 63-67). Die heterodimere PCB Lyase wird von den Genen cpcE und cpcE kodiert. Die Hämoxygenase wird durch hox1 und die Ferredoxin Oxidoreduktase durch das pcyA Gen kodiert. Das cpcA-Gen kodiert die α-Untereinheit CpcA und das cpcB-Gen die β-Untereinheit CpcB des Phycocyanins.

Trotz des großen Bedarfs an Fluoreszenzmarkern mit den besonderen spektralen Eigenschaften des Phycocyanins für die Zellbiologie und der Kenntnis der an der Biosynthese beteiligten Proteine sowie deren Gene ist es bisher nicht gelungen, Phycocyanin oder funktionell aktive Teile daraus - wie beispielsweise das Holo-CpcA oder das Holo-CpcB - rekombinant in Eukaryonten zu exprimieren.

In der Vergangenheit ist zwar die heterologe Expression des Holo-CpcA in *E. coli* gelungen (Tooley et. al. a.a.O.). Dazu wurden die Zellen mit zwei Plasmiden mit cpcA, cpcE und cpcF Gen bzw. hox1 und pcyA transfiziert. Nach Induktion der Genexpression erfolgte die Biosynthese des Holo-CpcA. Auch zeigte dieses Holo-CpcA vergleichbare spektrale Eigenschaften wie das native Holo-CpcA. Die Ausbeute an Holo-CpcA war aber gering, da nur etwa ein Drittel des verfügbaren Apo-CpcA tatsächlich zum Holo-CpcA umgesetzt wurde.

In ihren Untersuchungen haben Tooley *et al.* evaluiert, ob möglicherweise ein Mangel an verfügbarem endogenen Häm in *E. coli* für die reduzierte Konversion von Apo-CpcA zu Holo-CpcA ursächlich ist. Dazu haben sie den transformierten Zellen zusätzlich δ-Aminolävulinsäure angeboten. Die zusätzliche Gabe dieser Schlüsselverbindung des Häm-Stoffwechsels hat jedoch nicht zu einer Erhöhung der Holo-CpcA Ausbeute geführt.

Die Autoren spekulieren daher darüber, ob die für die Expression in *E. coli* ungünstige Codonverwertung ("Codon Usage") der Gene hox1, pcyA, cpcE und cpcF für die mangelhafte Ausbeute verantwortlich sein könnte. Des weiteren könnte eine ungleiche Anzahl der jeweils in einer *E. coli*-Zelle vorliegenden Plasmide - die entweder hox1 und pcyA oder aber cpcA, cpcE und cpcF tragen - eine Rolle spielen (siehe dazu auch Tooley *et al.* a. a.O. Seite 10564 rechte Spalte oben).

Auch andere Arbeitsgruppen haben bereits vor langer Zeit die Vorteile der Verwendung von Phycobiliproteinen als Marker für intrazelluläre Vorgänge in vivo erkannt und daher versucht, Systeme zur Bereitstellung von Holo-CpcA in Hefen zu etablieren. Dazu wurden beispielsweise die Gene cpcE und cpcF für die heterodimere PCB-Lyase sowie das cpcA-Gen zu Kodierung des Apo-α-CPCA in die Hefen kloniert. Durch eine anschließende Gabe exogenen PCB war beabsichtigt, ein funktionelles Holo CpcA - das heißt also einen funktionell aktiven Teil des Phycocyanins - in den Hefen zu generieren. Dieser Versuch der semisynthetischen Bereitstellung des Holo-CpcA in Hefen schlug jedoch fehl. (Schröder B.G. (1997) Doctorial Dissertation (University of California, Berkeley): Phycobiliprotein: Biosynthesis and Applications)

Die möglichen Ursachen für den fehlgeschlagenen Versuch, funktionell aktive Teile des Phycocyanins durch die Addition exogenen PCBs an rekombinant erzeugtes Apo-CpcA in Hefen zu generieren, wird von Schröder ausführlich diskutiert. So konnte er durch die Wahl geeigneter Versuchsbedingungen ausschließen, dass die fehlende Addition des PCB an CpcA durch einen möglicherweise fehlenden Import des externen PCB in die Zelle oder aber durch einen aktiven Export des zunächst importierten PCB aus der Zelle heraus bedingt war.

Er schloß aufgrund seiner Untersuchungen daher aus, dass die Additionsreaktion der in das Hefegenom einklonierten CpcE CpcF Lyase aufgrund einer unzureichenden Konzentration an PCB ausblieb. Er folgerte aus seinen Untersuchungen des weiteren, dass in der Hefe ein die Additionsreaktion hemmender Inhibitor vorhanden sein muß, und verweist für die Zukunft auf die Möglichkeit, Hefemutaten zu identifizieren, die diesen Inhibitor nicht aufweisen.

Ferner spekuliert Schröder auf die Möglichkeit, nach einer Aufklärung des vollständigen Biosynthesewegs die dafür relevanten Gene in die Hefe einzuklonieren und somit eine vollständige *in vivo* Biosynthese zu erreichen (siehe zu der Diskussion dieses Problems auch Schröder a. a. O. Seite 77-79). Eine eindeutige Erklärung für das Scheitern seiner Bestrebungen zur Bereitstellung einer semi-synthetischen Biosynthese bteibt Schröder aber schuldig.

In diesem Zusammenhang sei nochmals darauf hingewiesen, dass es Tooley *et al*. aber selbst nach der Aufklärung des Biosynthesewegs nicht gelungen ist, eine zufriedenstellende Expression in *E. coli* zu erreichen (siehe oben).

Vor dem Hintergrund dieses Stands der Technik ist es um so überraschender, dass nun erstmals die Transformation einer Eukaryontenzelle gelungen ist, die ein Holo-CpcA als funktionell aktives Teil eines Phycocyanins exprimiert. Dabei wird in diesem Zusammenhang unter einem "funktionell aktiven Teil" jedes Teil, Analogon oder Derivat des Phycocyanins verstanden, das zu mindestens einer der Phycocyanin α- oder β-Untereinheiten (oder Teilen davon) eine strukturelle Homologie aufweist und in seinen spektralen Eigenschaften im wesentlichen denjenigen Eigenschaften des nativen Phycocyanins entspricht.

In einer besonders bevorzugten Ausführungsform exprimiert die erfindungsgemäße Eukaryontenzelle das Holo-CpcA. Dazu kann die Eukaryontenzelle vorzugsweise mit den Genen cpcE und cpcF für die PCB-Lyase, dem hox1 für die Hämoxygenase, dem pcyA für die Ferrodoxin-Reduktase und dem cpcA für die α-Untereinheit transformiert sein. Damit kann eine vollständig endogene Biosynthese des Holo-CpcA in einem heterologen eukaryontischen Wirt erreicht werden.

Ebenso ist es aber auch möglich, das Holo-CpcA über einen semisynthetischen Biosyntheseweg bereitzustellen. Dazu kann es ausreichen, das CpcA oder CpcB - oder zumindest den jeweils für die Addition des PCB relevanten Teils davon - sowie die PCB Lyase in der Eukaryontenzelle zu exprimieren und PCB exogen hinzuzuführen. Alternativ können auch ein oder mehrere der anderen für die Biosynthese relevanten Gene entweder einkloniert werden oder aber ihr jeweiliges Genprodukt extern hinzugefügt werden. Damit ist es möglich - in Abhängigkeit von der jeweiligen konkreten Fragestellung des Versuchs ― das System gezielt zu induzieren. Mit der anschließenden Isolierung des Holo-CpcA oder Holo-CpcB aus der Wirtszelle steht das erfindungsgemäße "funktionell aktive Teil" des Phycocyanins bereit.

In dem Fall, dass sowohl das Holo-CpcA als auch das Holo-CpcB in der Zelle exprimiert werden - entweder vollständig endogen oder aber semisynthetisch wie oben beschrieben - können die beiden Untereinheiten dimerisieren und ein komplettes Phycocyanin-Monomer bilden. Erfindungsgemäß kann somit nicht nur ein Teil des Phycocyanins, sondern auch ein vollständiges Phycocyanin bereitgestellt werden.

Das erfindungsgemäß bereitgestellte Phycocyanin bzw. dessen funktionell aktive Teile weisen ein Glycosylierungsmuster auf, das sie von dem jeweils nativen Protein oder der entsprechenden Proteinuntereinheit unterscheidet. So ist beispielsweise das in einem eukaryontischen Wirt exprimierte CpcA an dem Threonin der Position 6, dem Serin an Position 10 und dem Serin an Position 162 der Aminosäuresequenz über eine O-Verknüpfung glykosyliert. Diese Glykosylierungen liegen im nativen CpcA nicht vor.

Ein besonderer Vorteil des erfindungsgemäßen Fluoreszenzproteins besteht darin, dass sein Emissionsspektrum im Vergleich mit seinen bisher bekannten Expressionsformen eine Rotverschiebung zeigt. Bei einem pH von 8,0 liegt das Emissionsmaximum des Holo-CpcA bei 643 nm und damit um 2 nm weiter im längerwelligen Bereich als beispielsweise das in *E.coli* exprimierte CpcA. Selbst wenn dieser Unterschied quantitativ nicht groß ist, kann er in der Praxis aber dazu dienen, dieses Signal von Signalen anderer Fluoreszenzfarbstoffe zu unterscheiden.

In einer besonders vorteilhaften Ausführungsform der Erfindung wird das Holo-CpcA in einer transformierten Hefezelle exprimiert. Dazu eignet sich insbesondere der unter der Aufnahmenummer DSM 16134 am 15. Januar 2004 bei der Deutschen Sammlung von Mikroorganismen und Zellkulturen GmbH (DSMZ) hinterlegte Hefestamm.

Es hat sich gezeigt, dass die heterologe Expression von Holo-CpcA nicht nur abhängt von der erfolgreichen Klonierung der für die Biosynthese erforderlichen Gene in die Wirtszelle, sondern auch durch eine geeignete Wahl der Kultivierungsbedingungen der Wirtszelle beeinflußbar ist. Dabei können insbesondere Kultivierungsbedingungen eine Rolle spielen, die die Konzentration an verfügbarem endogenen Häm in der Wirtszellen beeinflussen. So kann eine Stimulierung der Häm-Biosynthese oder aber eine Hemmung des zellulären Häm-Abbaus zu einer Erhöhung der endogenen Häm-Konzentration führen.

Die insgesamt 8 Schritte umfassende Biosynthese des Häms ist seit langem bekannt und die dafür in der Hefe verantwortlichen Gene sind bereits identifiziert (z.B. R. Labbe-Bois, P. Labbe: Tetrapyrrol and Heme biosysnthesis in the yeast *Saccharomyces cerevisiae.* In: H.A: Dailey (Ed.) Biosynthesis of Heme and Chlorophylls, Mc Graw-Hill, New York, 1990, 235-286 oder A. Chelstowska, J. Rytka: Heme bioysnthesis in the yeast *Saccharomyces cerevisiae.* Postpy Biochem. 39 (1993) 173-185). Die Regulationsmechanismen dieses Syntheseweges sind jedoch noch nicht vollständig verstanden. Es hat sich aber gezeigt, dass die Produktionsrate des Häms u.a. von der verwendeten Kohlenstoffquelle im Medium und der Sauerstoffverfügbarkeit während der Kultivierung abhängt. So führt die Gabe von Glucose zu einer Repression der Hämsynthese. Gleiches gilt für andere von der Hefe fermentierbare Kohlenstoffquellen wie z.B. Galactose. Die Gabe von nicht-fermentierbaren Kohlenstoffquellen wie Ethanol stimuliert dagegen im Vergleich zu Glucose den Hämstoffwechsel um das 2 - 3-fache (M. Hoffmann, M. Gora, Rythka J: Identification of rate-limiting steps in yeast heme biosynthesis. Biochemical and Biophysical research Communications 310 (2003) 1247-1253; Chelstowska (1993)).

Somit ist es für die erfindungsgemäße Bereitstellung des rekombinanten Phycocyanins bzw. dessen Teile vorteilhaft, die transformierten Hefezellen in einem Medium mit nicht-fermentierbaren Kohlenstoffquellen zu kultivieren, um die endogene Konzentration des verfügbaren Häms zu steigern.

Alternativ können in einer vorteilhaften Ausführungsform des erfindungsgemäßen Herstellungsverfahrens Inhibitoren des Hämabbaus dem Kulturmedium zugesetzt oder in den Wirtsorganismus kloniert werden.

Bei dem Enzym HMX1 handelt es sich um ein Homolog der Hämoxygenase, das der unveränderten Hefe fehlt (Protchenko O., Philpott C.C. (2003) Regulation of Intracellular Heme Levels by HMX1, a Homologue of heme Oxygenase, in *Saccaromyces cerevisiae. JBC* 278:36582-36587). Da dieses Enzym über Eisen gehemmt wird, besteht die Möglichkeit, dieses Enzym in einem konkreten Kulturmedium zu hemmen, z. B. durch die Gabe von Eisen.

Die Konzentration an verfügbarem Häm kann erfindungsgemäß ebenso durch eine Überexpression der Schlüsselenzyme der Hämsynthese erfolgen. Dazu kann die Porphobilinogen Synthase, die Porphobilinogen Deaminase oder die Uroporphyrinogen III Decarboxylase überexprimiert werden (Hoffmann *et al* (2003) a.a.O.).

Ebenso kann es - bei der Verwendung des ADH-Promotors als Promotor der einklonierten Gene - vorteilhaft sein, die Aktivität des Promotors z.B. durch eine Erhöhung der Glucose-Konzentration zu steigern. Die Erhöhung der Glucosekonzentration zur Steigerung der Promotoraktivität steht zwar zunächst im Konflikt mit der damit verbundenen Repression des Hämstoffwechsels. Es hat sich aber experimentell bestätigt, dass insbesondere eine Erhöhung der Glucosekonzentration im Medium von dem bei der Kultivierung von Hefen üblichen 2 % auf beispielsweise 13 % zu einer optimierten Ausbeute von rekombinantem Holo-CpcA mit einer Steigerung des Fluoreszenzsignals um das mehr als 7-fache führt (siehe unten).

In einer besonders vorteilhaften Ausführungsform wird das Kulturmedium mit 2 % Ethanol versehen. Es ist bekannt, dass Ethanol einerseits als nicht fermentierbare Kohlenstoffquelle den Hämstoffwechsel induziert (Hoffmann et al (293) a.a. O.), aber andererseits auch die Aktivität des ADH Promotors fördert (Ruohonen, L., Aalto, M.K., Keränen, S.: Modifikations to the ADH1 promotor of Saccharomyces ceriviseae for efficient production of heterologous proteines. Journal of Biotechnology 1995 (39) 193-203)a.a.O.).

Erfindungsgemäß kann alternativ oder ergänzend vorgesehen werden, den Abbau des rekombinanten Phycocyanins in der Zelle bzw. dessen Teile zu hemmen oder zu verzögern. Da ein Stickstoffmangel einen verstärkten Abbau der Phycocyanin Hexamere begünstigt (Grossman AR (1990) Chromatic adaptation and the events involved in phycobilisome biosynthesis. *Plant, Cell and Environment* 13:651-666), kann eine Supplementienrng mit Stickstoff dem Phycocyanin-Abbau entgegenwirken.

Wie bereits erwähnt, wird in einer Ausführungsform der Erfindung eine Hefezelle transformiert. Dazu ist es vorteilhaft, die einklonierten PC-Gene für die Expression in der Hefe zu optimieren, indem zunächst der GC-Gehalt der Gene auf einen durchschnittlichen Wert von 45% gebracht wird. So kann die genetische Stabilität und die Halbwertzeit der mRNA im Wirtsorganismus verlängert werden.

Darüber hinaus kann die Effizienz der Expression noch gesteigert werden, indem die PC-Gene hinsichtlich ihrer Codon-Verwertung ("Codon Usage") auf den jeweiligen Wirtsorganismus eingestellt werden. In einer besonders bevorzugten Ausführungsform wird die Eukaryontenzelle mit einem oder mehreren Plasmiden, die eine oder mehrere der Oligonukleotisequenzen der SEQ. ID. No. 6 - 10 transformiert.

Für die erfolgreiche Transformation des eukaryontischen Wirts werden die ausgesuchten PC-Gene zunächst in einen Klonierungsvektor kloniert und können anschließend über einen Integrationsvektor in das Genom der Wirtszelle stabil integriert werden. Die Gene können dabei auf einen oder mehrere Klonienrngsvektoren verteilt werden. In einer besonders bevorzugten Ausführungsform der Erfindung ist die Klonierung aller 5 PC-Gene in nur ein Plasmid erfolgt.

Sofern die konstitutive Expression eines Gens auf dem Vektor oder in dem. Genom gewünscht ist, kann diesem Gen ein konstitutiver Promotor - beispielsweise ein ADH-Promotor - vorgeschaltet sein. Es sind jedoch auch induzierbare Promotoren, wie beispielsweise der GAL-Promotor möglich. Die Promotoren können sowohl jedem einzelnen Gen vorgeschaltet sein, als auch die gesamte Gruppe der Gene gemeinschaftlich regulieren.

In einer bevorzugten Ausführungsform wurde ein ADH Promotor gewählt, um die Expression der PC-Gene hox1, cpcE, pcyA und cpcF zu ermöglichen. Der ADH Promotor ist dabei jedem Gen jeweils vorgeschaltet. In dieser Ausführungsform wird das cpcA Gen über den GAL-Promotor reguliert. Damit kann ein induzierbares System für die Biosynthese des Phycocyanins oder des Holo-α-Phycocyanins geschaffen werden.

Selbstverständlich ist es alternativ auch möglich, das cpcA-Gen unter die Kontrolle des ADH. Promotors und ein oder mehrere der übrigen Gene unter die Kontrolle des GAL Promotors oder eines beliebigen anderen Promotors zu stellen.

Sofern ein ADH1-Promotor verwendet wird, ist es von Vorteil, den ADH Promotor nicht in seiner ursprünglichen Länge von etwa 1500 bp zu verwenden, da sich erfahrungsgemäß die Aktivität des ADH1 Promotors über die Zeit reduziert. So konnte beobachtet werden, dass der vollständige Promotor im späten exponentiellen Wachstum der Zellen abschaltet.

Nach Untersuchungen von Ruohonen *et al.* ist diese Eigenschaft des ADH1 Promotors auf ein stromaufwärts gelegenes 700 bp Fragment zurückzuführen. Die Deletion dieser Region hin zu einen "mittleren Promotor" von etwa 700 bp ist dagegen für eine hohe und stabile Expression der klonierten Gene geeignet. Die Publikation von Ruohonen *et al.* wird hinsichtlich der Struktur und Lage eines erfindungsgemäß verwendbaren ADH1 Promotors vollumfängtich in Bezug genommen.

In einer bevorzugten Ausführungsform wird ein ADH1 Promotor mit der Nukleotidsequenz gemäß SEQ. ID. No. 5 eingesetzt.

Da eukaryontische mRNA im Gegensatz zu prokaryontischer mRNA nicht polycistronisch ist, müssen die einklonierten Gene jeweils mit einer Terminationssequenz versehen sein. In einer vorteilhaften Ausführungsform wird die. Terminationssequenz nach Guo und Sherman eingesetzt (Guo, Z., Sherman, F. Signals sufficient for 3' end formation of Yeast mRNA. Mol Cell Biol 1996 (16), 2772-2776). Besonders bevorzugt ist die unter Ziffer 3 dargestellte Terminationssequenz SEQ. ID. No. 13.

### AUSFÜHRUNGSBEISPIEL

### 1. Klonierungsstrategie

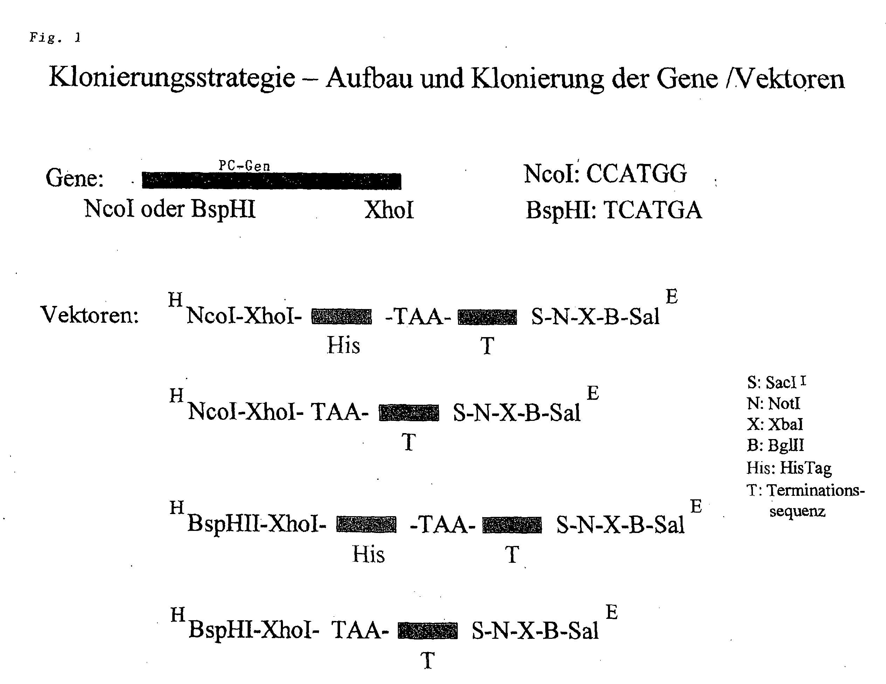

### (siehe auch Fig. 1 "Klonierungsstrategie: Aufbau und Klonierung der Gene/Vektoren")

### 1.1. Klonierung und Synthese der Gene:

Da die Umgebung des Startcodons erhalten bleiben soll, kommen folgende Enzyme in Frage:

| Enzym | Erkennungsseq. | Isoschizomere | kompatible Enden | Recut |
|---|---|---|---|---|
| BspHI | T CATG A A GTAC T | | Ncol AfIIII (A CATGT) | Nlalll |
| Ncol | C\|CATG G G GTAC C | | Afllll BspHl, Pcil | Nlalll |

BspHI und NcoI sind kompatibel und schneiden nicht in den Gensequenzen.

Die Gene wurden über 5' Ncol oder BspHI und 3' Xhol in die Vektoren kloniert. Hierzu erhielten die Gene 5' die jeweils nach Umgebung des Startcodons passende Restriktionsschnittstelle. Am 3'-Ende wurde die Restriktionsschnittstelle für Xhol angefügt. Hier wurde darauf geachtet, dass möglichst wenige Aminosäuren durch die Klonierung zusätzlich in die Sequenz eingefügt wurden. Außerdem durfte durch das Anfügen der Xhol-Schnittstelle kein Frameshift entstehen. Dem wurde durch das Einfügen der Basen AA vor der Schnittstelle und dem Anhängen eines A nach der Schnittstelle Rechnung getragen. Hierdurch wurde an jedes Gen die Aminosäuresequenz NSR angehängt, bei der sichergestellt wurde, dass sie nicht durch seltene Codons codiert wird.

### 1.2. Klonierung und Synthese der Vektoren:

Die Vektoren sollen ein nachfolgendes Ausschneiden des gesamten Gens wahlweise mit und ohne His-Tag erlauben, um einen Nachweis der Expression der Einzelgene zu ermöglichen. Zur Klonierung der Gene hintereinander wurde der HisTag jedoch weggelassen, um eventuelle Probleme mit der zusätzlichen Sequenz zu vermeiden.

Somit enthalten die Vektoren Ncol-Xhol oder BspHI-Xhol je mit und ohne HisTag sowie mit und ohne Terminationssequenz. Durch das vorliegende Design der Vektoren können die Gene in diese einkloniert und danach mit/ohne HisTag, Stopcodon und Terminationssequenz ausgeschnitten und weiter kloniert werden.

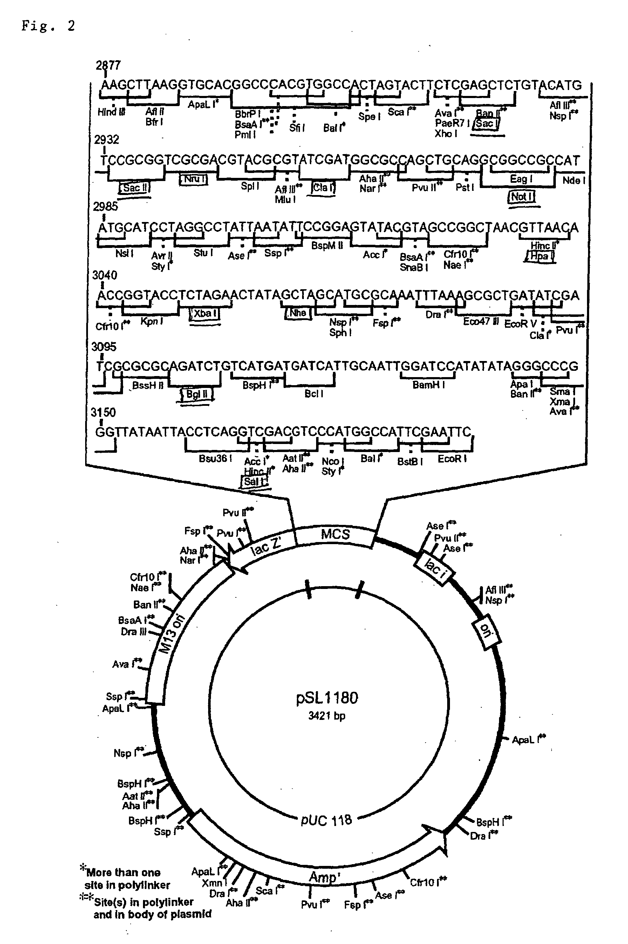

Als Ausgangsvektor, in den die synthetisierten mcs und die Gene hintereinander einkloniert wurden, diente pSL1190 (s. Karte von pSL 1180 (Fig. 2); die Vektoren pSL1190 und pSL1180 unterscheiden sich lediglich in der Orientierung der mcs). Die bei der Synthese der vier verschiedenen mcs-Sequenzen berücksichtigten Restriktionsschnittstellen sind in der Figur durch Kästen kenntlich gemacht - so sind mehr Möglichkeiten bei der Klonierung vorhanden. Die letztlich bei der Klonierung der PC-Gene verwendeten Restriktionsschnittstellen sind in der Figur zusätzlich durch einen den jeweiligen Kasten unterlegenden Strich markiert.

Beim Design der mcs für die Vektoren wurde auf folgende Dinge geachtet:
die verwendeten Enzyme dürfen nicht in den Promotorsequenzen schneiden
sie dürfen nicht im Rückgrat des Vektors schneiden
der Abstand zwischen den Restriktionsschnittstellen muss groß genug sein, damit benachbarte Enzyme beide schneiden können
die mcs müssen in den pSL1190 kloniert werden können - hierzu werden die Enzymschnittstellen EcoRl und Hindlll an den Enden der mcs vorgesehen
es müssen Schnittstellen zur Integration der gesamten PC-Kassette (alle 5 Gene hintereinander kloniert) in den Vektor pRS306 vorliegen (hier: Sacl/Sall)
In dem Vektor pRS306 muss die Möglichkeit zur Linearisierung im URA3-Gen (Selektionsmarker) bestehen, damit der Vektor integrieren kann und die Kassette genomisch integriert vorliegt.

Die Sequenzen der mcs der Vektoren sind beigefügt (SEQ. ID. No. 1 - SEQ. ID No. 4).

Aus den Vektoren 656 und 660 (pSL1190 mit synthetisierter mcs, die BspHlsite enthält) müssen, um eine Klonierung mit BspHl durchführen zu können, drei Schnittstellen dieses Enzyms aus dem Rückgrat des Vektors entfernt werden. Hierzu wird der Quik Change Multi Site Directed Mutagenesis Kit von Stratagene eingesetzt und der Erfolg des Experimentes durch Restriktionsanalysen der entstandenen Vektoren verifiziert.

Entstandene Klone bei der Vektorklonierung: (die weiter verwendeten Klone sind **fett** gekennzeichnet)

| Klon-Nr.: B | Klonbeschreibung |
|---|---|
| 647 | GeneArt mcs-A = 03-200; NcoI/His-Tag in PCR-Script; **647** |
| 648 | GeneArt mcs-B = 03-201; BspHI/His-Tag in PCR-Script; **648** |
| 649 | GeneArt mcs-C = 03-215; NcoI/ohne His-Tag in PCR-Script; **649** |
| 650 | GeneArt mcs-D = 03-216; BspHI/ohne His-Tag in PCR-Script; **650** |
| 651 bis 653 | GeneArt mcs = 03-199 in pRS306; **651** |
| 654, 655 | GeneArt mcs-A = 03-200 = NcoI/His-Tag in pSL1190, **654** |
| 656, 657 | GeneArt mcs-B = 03-201 = BspHI/His-Tag in pSL1190 **656** |
| 658, 659 | GeneArt mcs-C = 03-215 = NcoI/ohne His-Tag in pSL1190; **658** |
| 660, 661 | GeneArt mcs-D = 03-216 = BspHI/ohne His-Tag in pSL1190; **660** |
| 682 bis 689 | QuikChange mit B656 zur Entfernung BspHI sites; **684, 685** |
| 690 bis 697 | QuikChange mit B660 zur Entfernung BspHI sites; **690, 693** |

### 1.3. Wahl und Klonierung des ADH Promotors:

Der ADH-Promotor (Alkohol-Dehydrogenase) ist in vielen Klonierungsvektoren vorhanden und führt dort zu einer konstitutiven Expression nachgeschalteter Gene. Oftmals ist jedoch bei seiner Verwendung ein Rückgang der Expressionsrate des klonierten Gens bis hin zu einem kompletten Abschalten der Expression nach etwa 3 Tagen festzustellen.

Es hat sich aber gezeigt, dass ein ein 700 bp langes Stück des originären (kompletten) Promotors umfassender ADH Promotor zu einer Expression der klonierten Gene während des gesamten Wachstumszyklus der Hefe führt (Ruohonen et al. (1995) a.a.O.).

Dieses Stück des Promotors war der Ausgangspunkt für den erfindungsgemäß verwendeten ADH-Promotor, wobei allerdings bei der Klonierung des Promotors die Primer für die PCR so gewählt wurden, dass ein ADH-Fragment von insgesamt 719 bp entstand (siehe SEQ. ID. No. 5).

Die Synthese des ADH Promotors wurde mittels PCR aus dem Genom von *S. cerevisiae* Y190 durchgeführt. Die Klonierung erfolgte in einer Drei-Fragmente-Ligation mit dem fraglichen Gen und dem Vektor zusammen. Hierfür wurde immer der gleiche reverse-Primer eingesetzt, der eine Ncol-Schnittstelle besitzt, da Ncol und BspHl kompatibel sind. Der forward-Primer besitzt jeweils eine andere angehängte Restriktionsschnittstelle, die je nach Klonierungsschritt ausgewählt wird.

Sequenzen der verwendeten Primer (die Restriktionsschnittstelle ist jeweils unterstrichen): Die zusätzlichen Basen werden benötigt, damit das Enzym das PCR-Fragment schneiden kann:

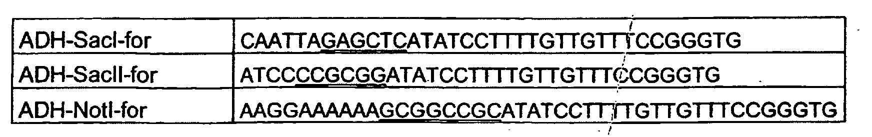

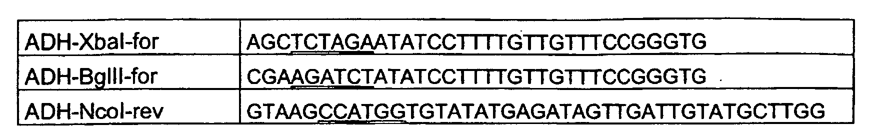

### 2. Optimierung der Codon-Usage

Die Codon-Usage wurde für die Häufigkeit der Codonnutzung (Codon-Usage) von *S. cerevisiae* optimiert. Der GC-Gehalt wurde, wenn möglich, auf einen durchschnittlichen Wert von ca. 45 % eingestellt, um die genetische Stabilität der mRNA-Halbwertzeit zu steigern.

Darüber hinaus wurden folgende für die Klonierung vorgesehenen Restriktionsschnittstellen aus den Genen entfernt:

| **Gen** | **Restriktionsenzym** | **Position (bp)** |
|---|---|---|
| CpcA | Kpnl | 312 |
| CpcE | Nhel | 779 |
| | Ybal | 493 |
| | Hpal | 366 |
| | Hpal | 549 |

Es wurden für die Klonierung der für die Expression des Holo-CpcA erforderlichen Gene ("PC-Gene") folgende Sequenzen verwendet:

| **Gen** | **SEQ. ID. No.** |
|---|---|
| cpcA | SEQ. ID. No. 6 |
| cpcE | SEQ. ID. No. 7 |
| cpcF | SEQ. ID. No. 8 |
| hox1 | SEQ. ID. No. 9 |
| pPcyA | SEQ. ID. No. 10 |
| cpcB | SEQ. ID. No. 11 |

Sofern ein Holo-CpcB exprimiert werden soll, kann alternativ zum cpcA das cpcB-Gen einkloniert werden (SEQ. ID. No. 11). Das codiert eine Aminosäuresequenz gemäß SEQ. ID. No. 12.

### 3. Wahl der Terminationssequenz

Da eukaryotische mRNAs im Gegensatz zu prokaryontischen nicht polycistronisch sind, muß eine Termination nach jedem Gen stattfinden. Guo und Sherman (1996, a.a.O.) beschreiben eine synthetische DNA-Sequenz, die für die Bildung eines 3'-Endes ausreicht und aus "efficiency element", "positioning element" und "poly(A) region" besteht.

Für unsere Zwecke wurde die Xbal-Schnittstelle entfernt und die Spacer-Region zwischen "efficiency element" und "positioning element" wie folgt gewählt: ACTCTGTAGA (statt ACTGTCTAGA; Xbal-Schnittstelle (SEQ. ID. No. 13)).

### 4. Klonierung der Einzelgene und Test der Expression der Einzelgene

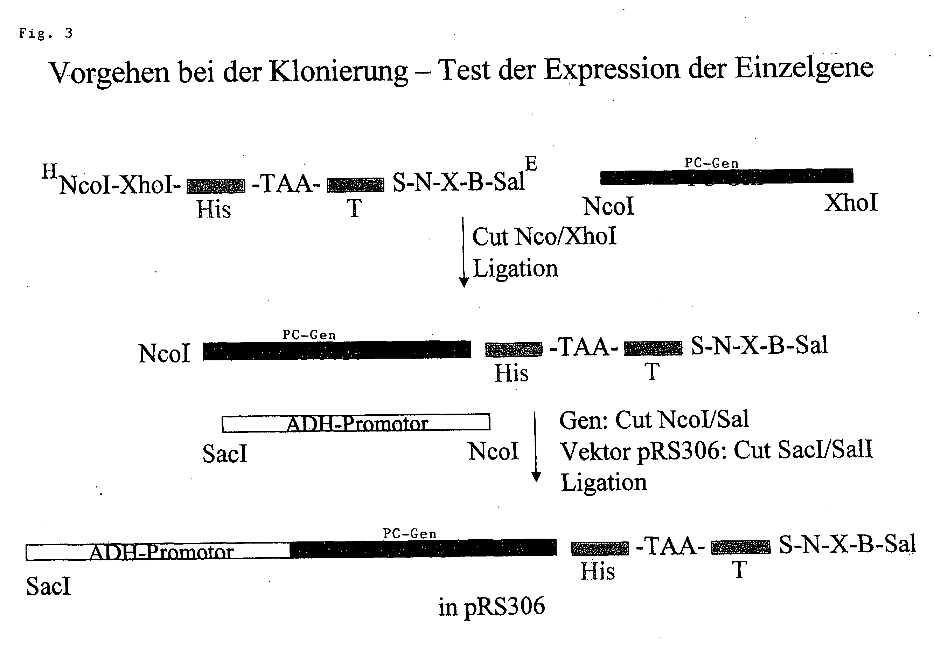

Das Vorgehen bei der Klonierung der Einzelgene sowie für den Nachweis ihrer erfolgreichen Expression ist schematisch in Fig. 3 dargestellt. Die Darstellung bezieht sich exemplarisch auf den Klon B654:pSL1190 mit Ncol-Schnittstelle und HisTag.

Die Linearisierung des fertigen Vektors erfolgte mit Stul. Die Transformation erfolgte in den Hefestamm Y190 mit URA Defizienz.

Zunächst wurden Vortests mit zwei anderen Fluorophoreh durchgeführt, um zu testen, ob eine Expression der Gene hinter dem gewählten ADH-Promotor und mit der eingesetzten Terminationssequenz erfolgt.

Für diesen Nachweis wurden die Gene einzeln jeweils mit dem ADH-Promotor in den Vektor pRS306 kloniert. Dabei handelt es sich um einen Integrationsvektor, so dass die Gene anschließend in das Genom der Hefe integriert werden können. Damit entspricht die Situation im wesentlichen dem angestrebten Ziel, nämlich dass die fünf für das Holo-CpcA relevanten Gene ("PC-Gene") hintereinander liegen. Als Schnittstellen zur Integration des Gens mit Promotor in den Vektor pRS306 wurde Sacl/Sall gewählt, da so am meisten Fremdsequenz in die Hefe eingebracht werden kann.

Hierzu wurden die Gene zuerst in den passenden Vektor (pSL1190 mit BspHI oder Ncol-site und HisTag) kloniert, um anschließend in einer Drei-Fragmente Ligation mit dem ADH-Promotor ligiert zu werden.

Zur Durchführung der Drei-Fragmente-Ligation wurden die eingesetzten DNA-Mengen mittels Agarosegel mit mitgeführtem Marker bestimmt. Danach wurden die eingesetzten DNA-Mengen auf molarer Ebene ausgerechnet. Dem Vektor wurden 25 fmol und den beiden Inserts je 125 fmol eingesetzt.

Das Zellpellet nach der Kultivierung von Protein-exprimierenden Hefen wurde mit *glass beads* und Vortexer aufgearbeitet. Hierbei wurden denaturierende Bedingungen gewählt. Bei der Aufreinigung des HisTag tragenden Proteins wurden die Vorschriften des Herstellers der Ni-NTA-Säulchen eingehalten.

### 5. Klonierung der Gene für das Holo-CpcA hintereinander

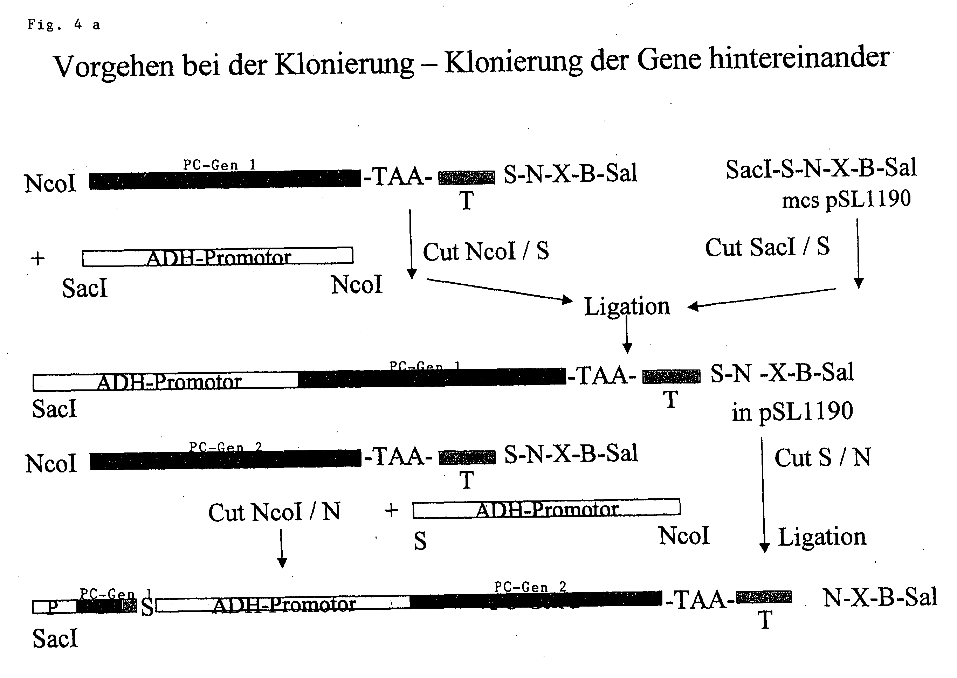

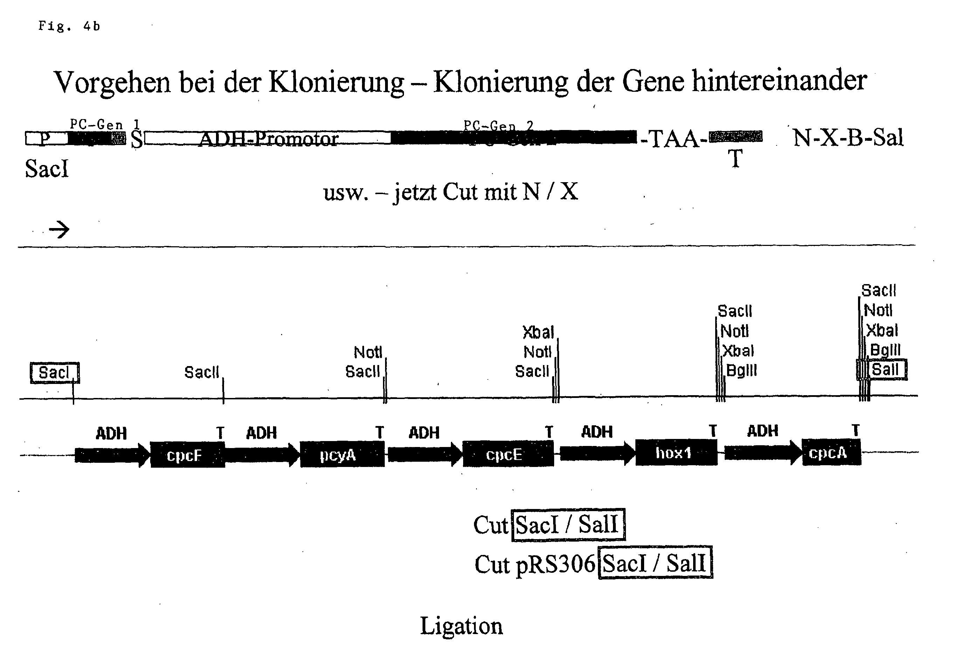

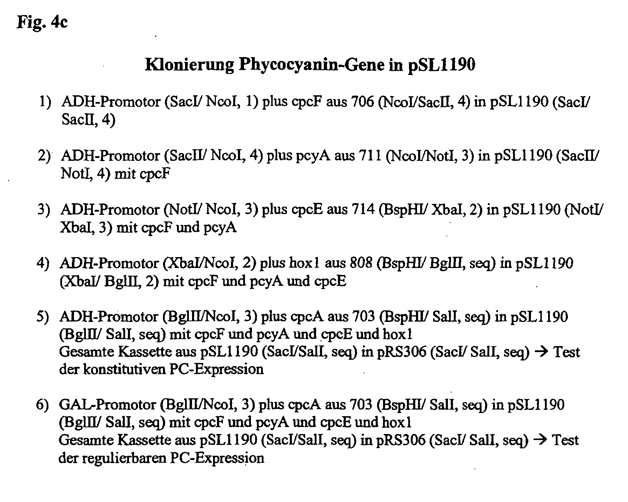

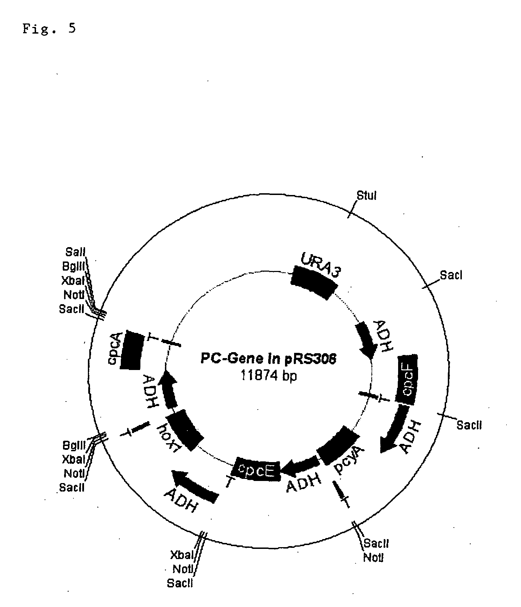

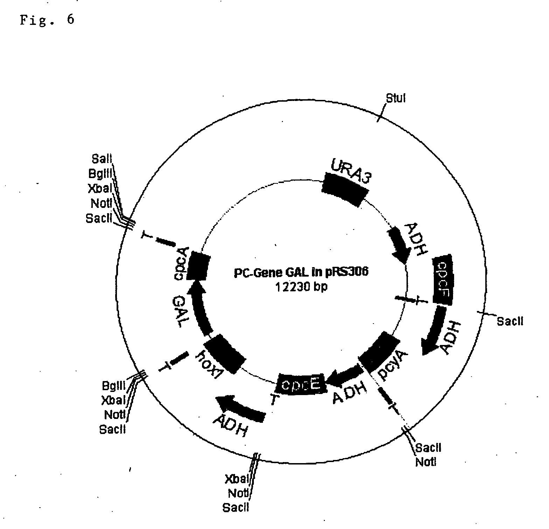

Das Vorgehen für die Klonierung der PC-Gene hintereinander ist schematisch in der Fig. 4a bis 4c dargestellt. Die Karte des Vektors mit den einklonierten PC-Genen zeigt Fig. 5. Die Sequenzdaten für den Vektor enthält SEQ. ID. No. 14. Sofern dem CpcA der Gal-Promotor vorgeschaltet ist, zeigt der Vektor einen Aufbau nach Fig. 6.

Die Gene wurden in pSL1190 mit synthetisierter mcs ohne HisTag kloniert. Danach wurden diese Gene mit passenden Enzymen herausgeschnitten und zusammen mit dem ADH-Promotor hintereinander in den Vektor pSL1190 gesetzt. Dabei wurde der Erfolg der Klonierungen mittels Bakterienkolonie-PCR für das jeweilige Gen nachgewiesen und die Identität der isolierten DNA wurde durch Testcuts verifiziert. Zusätzlich wurde der Promotoranteil der jeweils entstandenen Vektoren sequenziert (AGOWA), da dieser durch PCR erzeugt wurde (variierender forward primer mit verschiedenen Restriktionsenzymen) und Fehler vorhanden sein könnten. Für diese PCR-Anwendungen wurde die proof-reading-Polymerase Pfu (Promega) eingesetzt.

Nachdem die Gene hintereinander in dem Vektor pSL1190 vorlagen, wurde die gesamte Kassette über die Restriktionsschnittstellen Sacl/Sall in den Integrationsvektor pRS306 kloniert. Bei diesem Klonierungsschritt wurden das cpcA-Gen mit passenden Primem und der Vektor pRS306 mittels einer PCR mit Primem, die ein Stück aus dem URA3-Selektionsmarker amplifizieren, in den transformierten Bakterienkolonien nachgewiesen.

Sequenz der zum Nachweis der klonierten Gene/Integrationsvektor pRS306 verwendeten Primer:

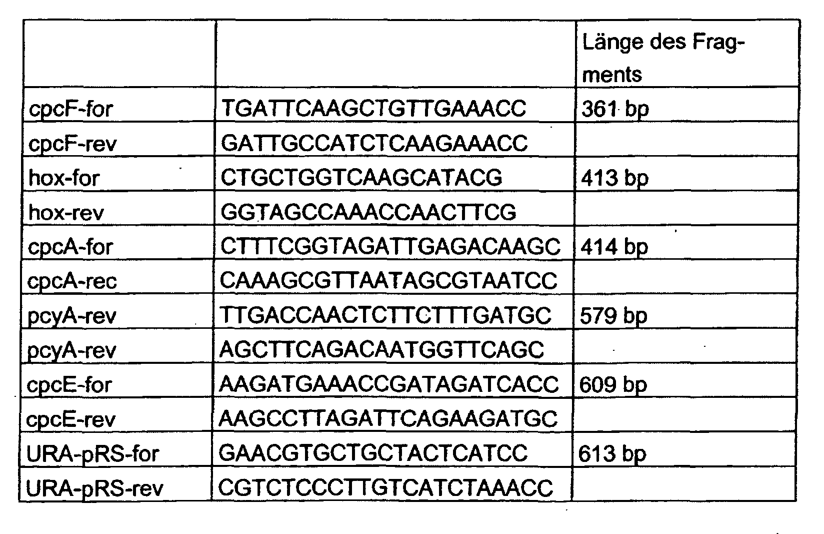

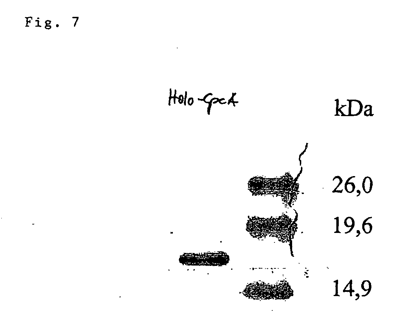

Um das Holo-Protein mittels Westem-Blot und Immunodetektion gegen den HisTag nachweisen zu können, wurde das cpcA-Gen mit HisTag hinter die Kassette mit den restlichen Genen gesetzt und dann diese vollständige Kassette in pRS306 kloniert. Nach der Transformation mit nachfolgender Expression des Proteins in der Hefe wurde das Protein aufgereinigt und in einem Westem Blot dargestellt (Fig. 7).

### Western Blot:

Für den Westem Blot wurde als primärer Antikörper der *Mouse Anti Histidine Tag* (Serotec) 1:250 eingesetzt. Als sekundärer Antikörper diente *Goat Anti Mouse Alkaline Phosphatase* (Dianova) 1:5000. Die Detektion 'erfolgte mit BCIP/NBT-Blue Liquid (Sigma).

Als Marker wurde der prestained SDS-Gel Marker (Invitrogen) verwendet.

### 6. Spektrum des erfindungsgemäßen Holo-CpcA

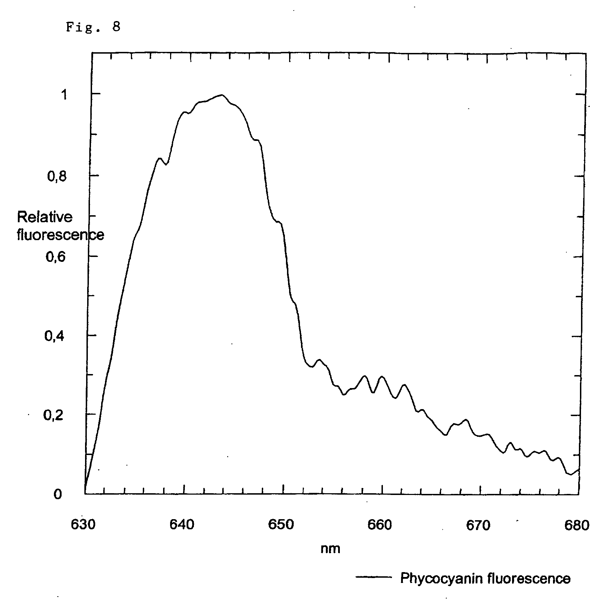

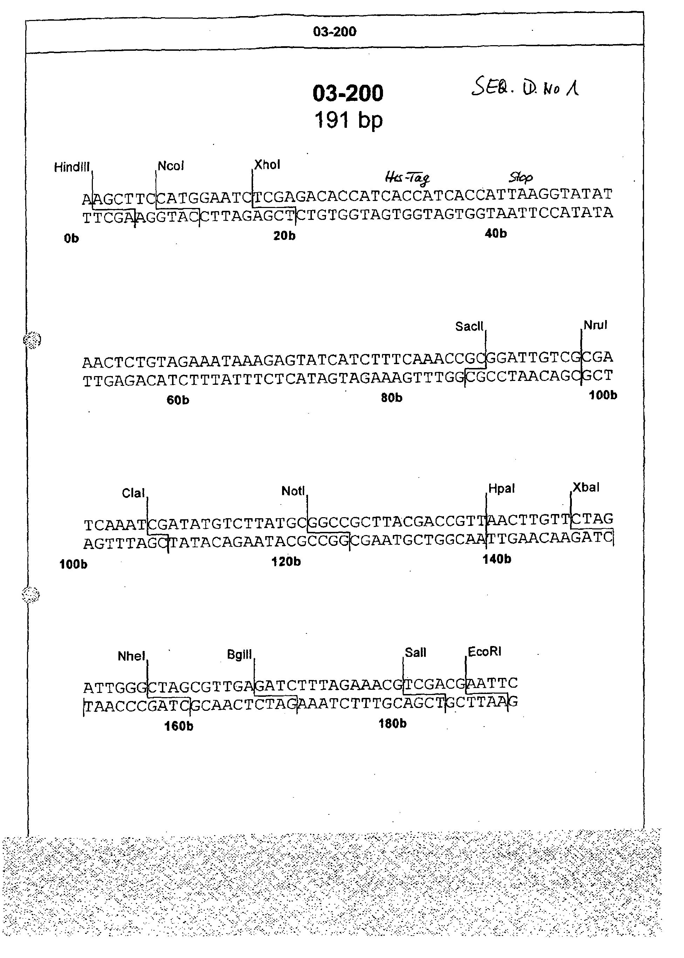

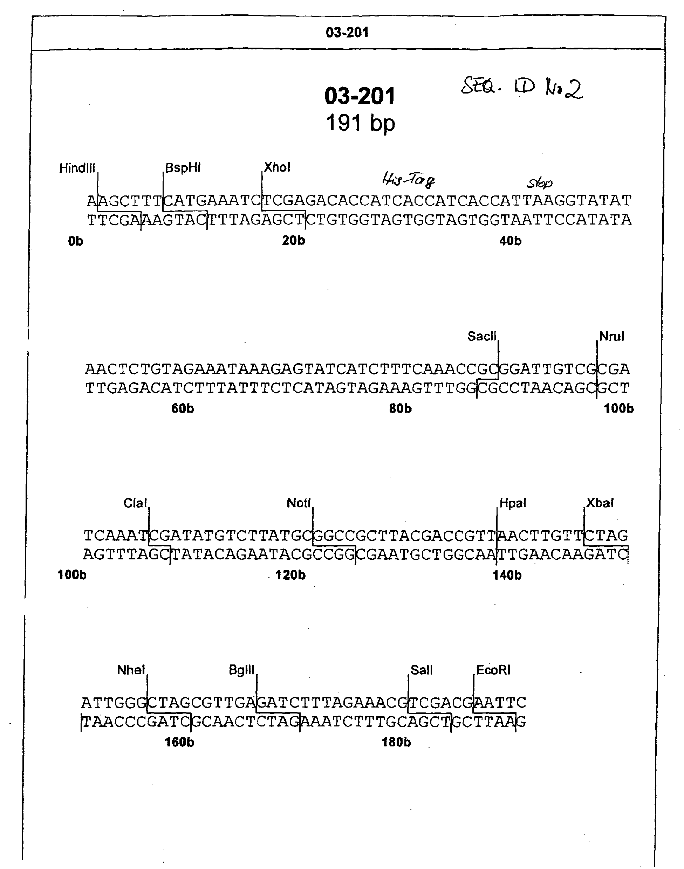

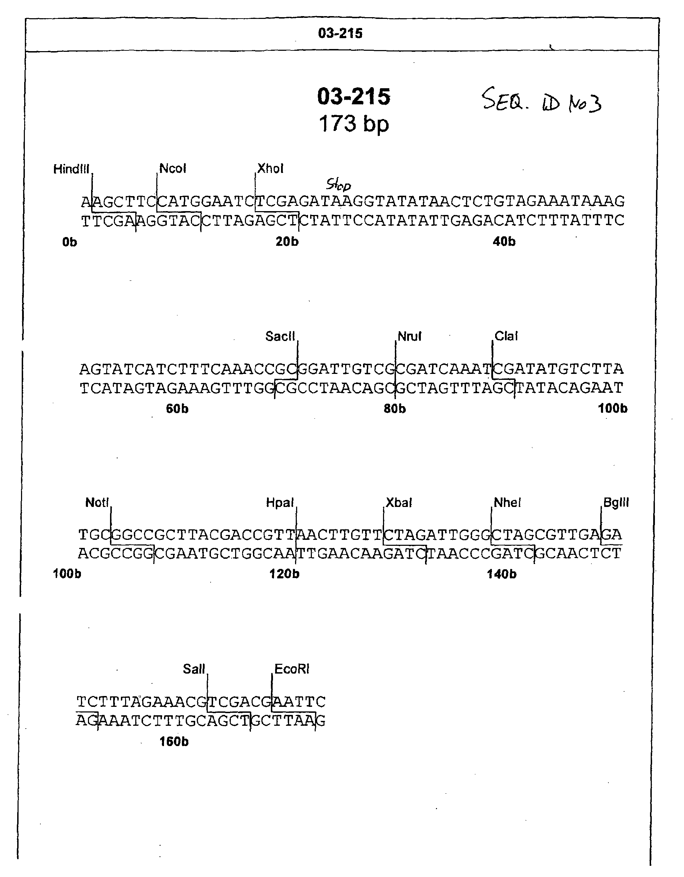

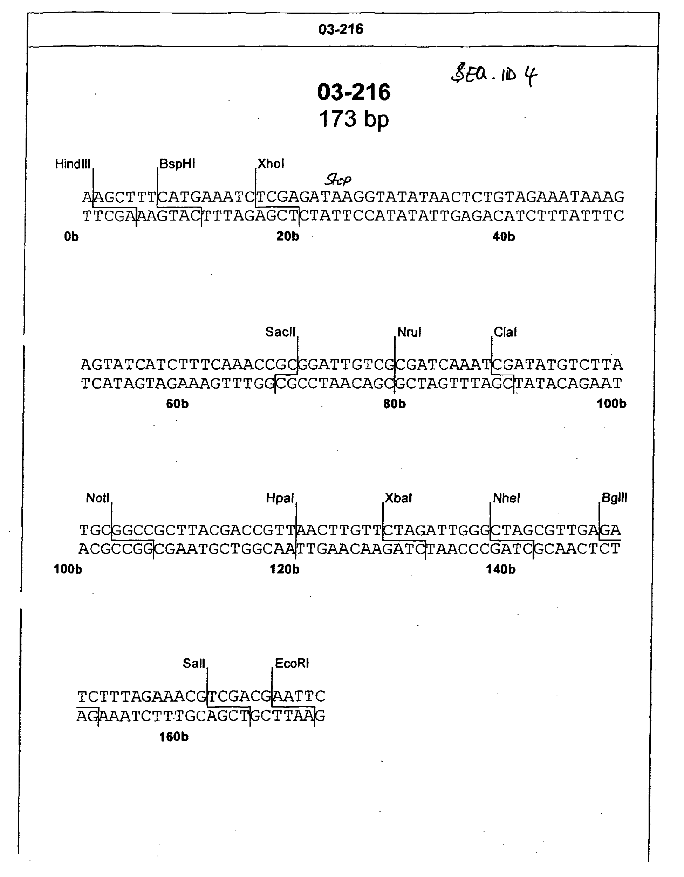

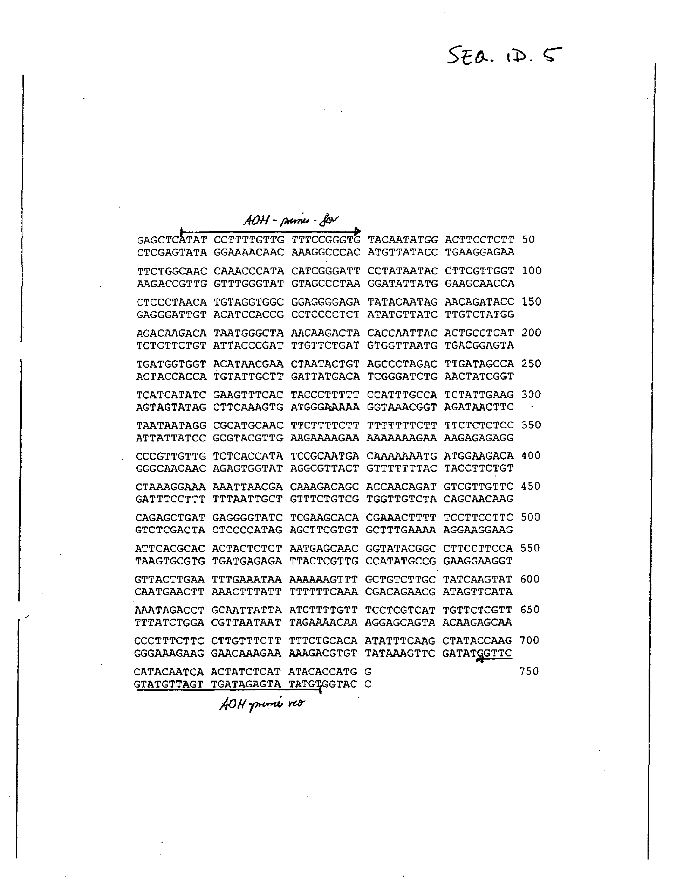

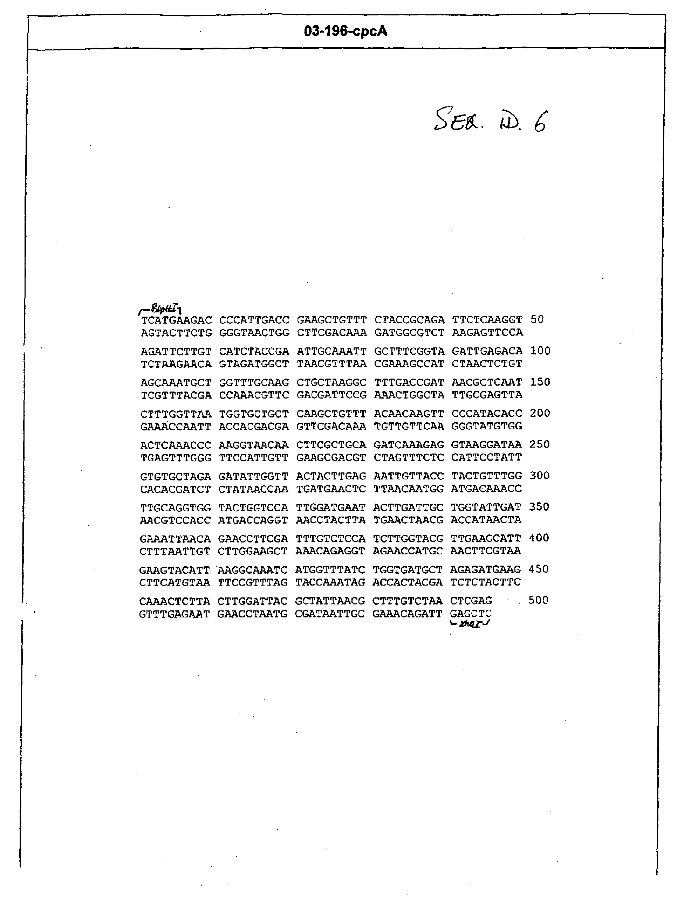

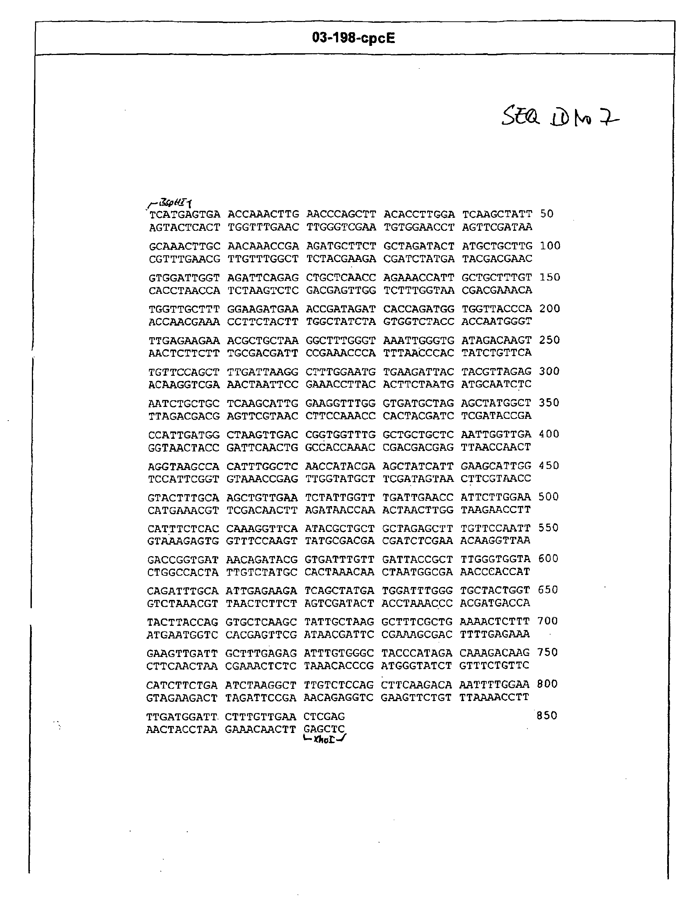

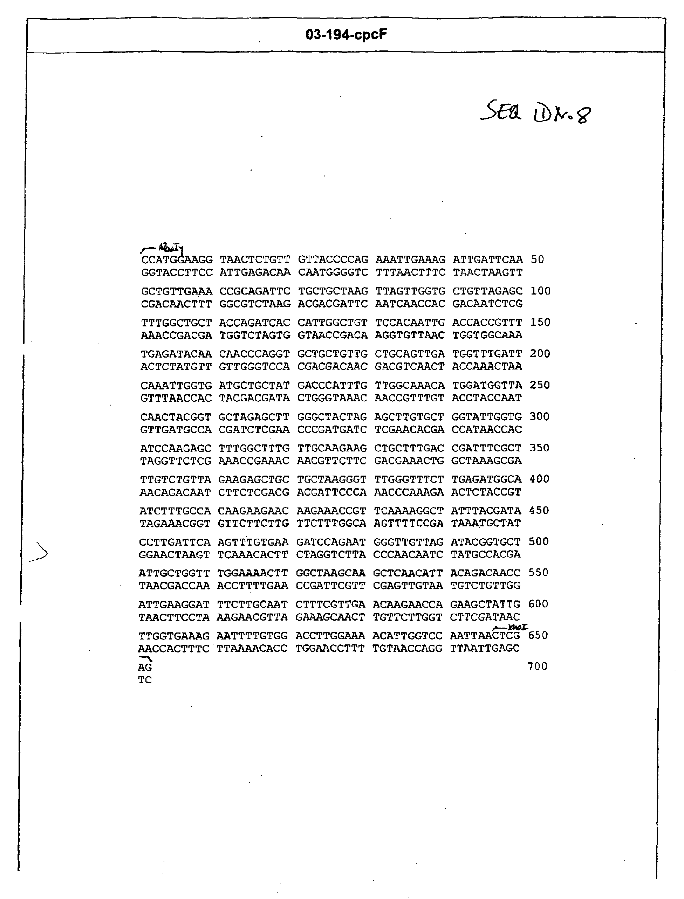

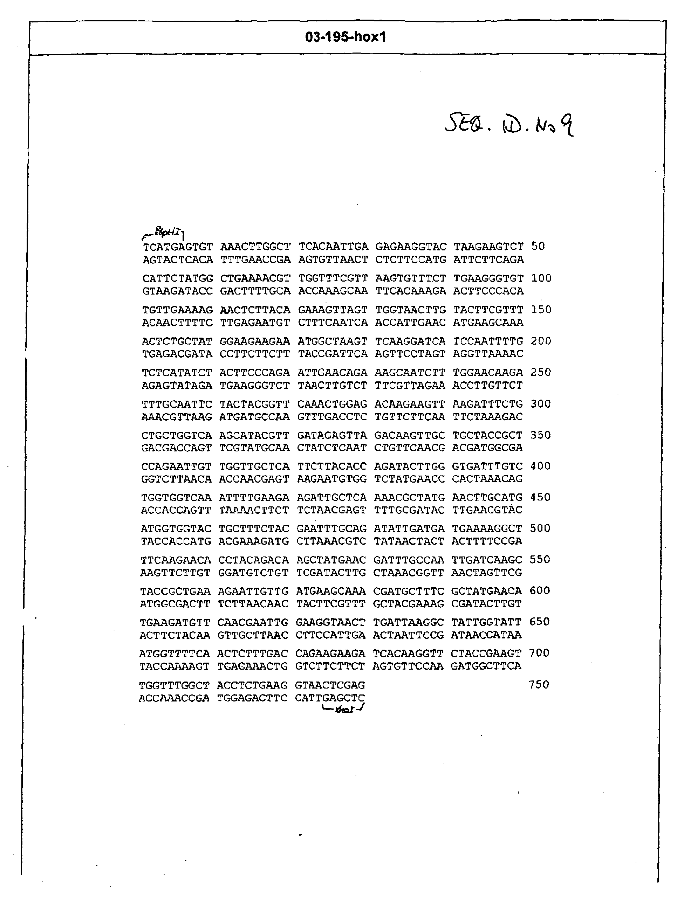

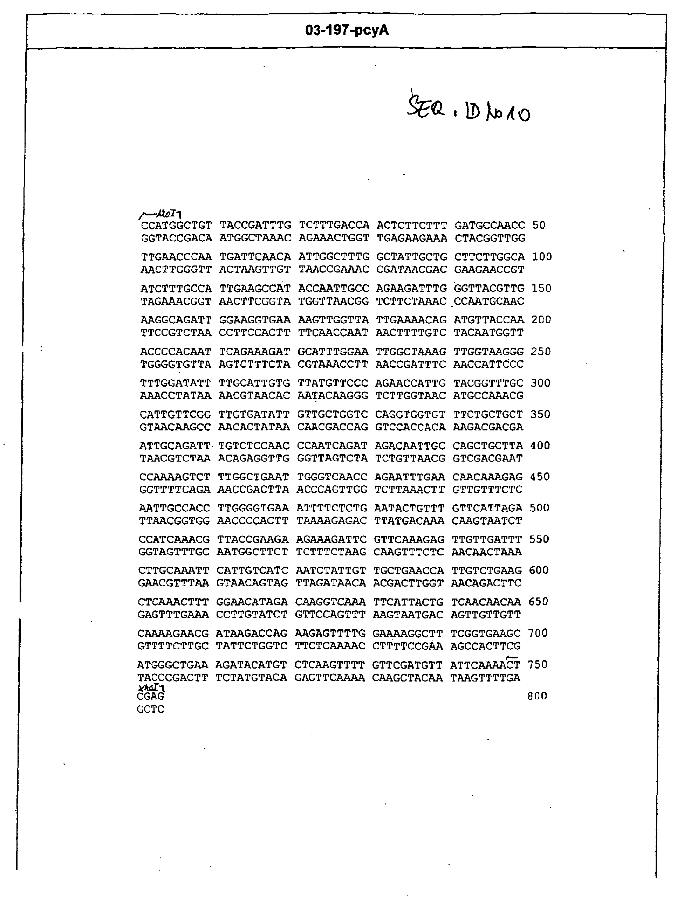

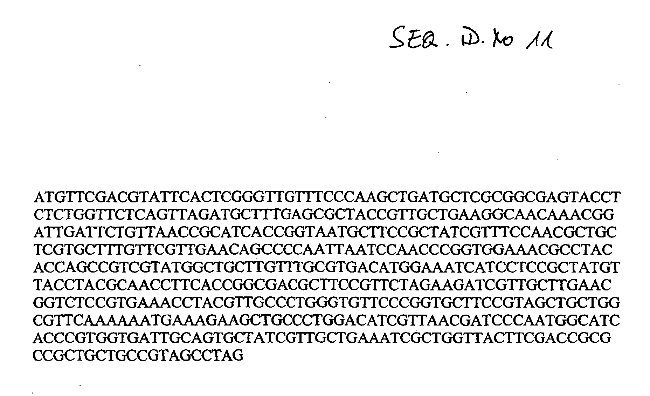

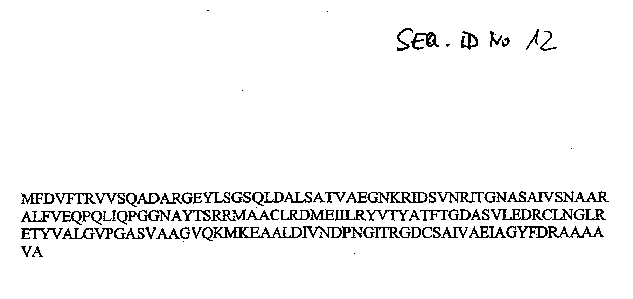

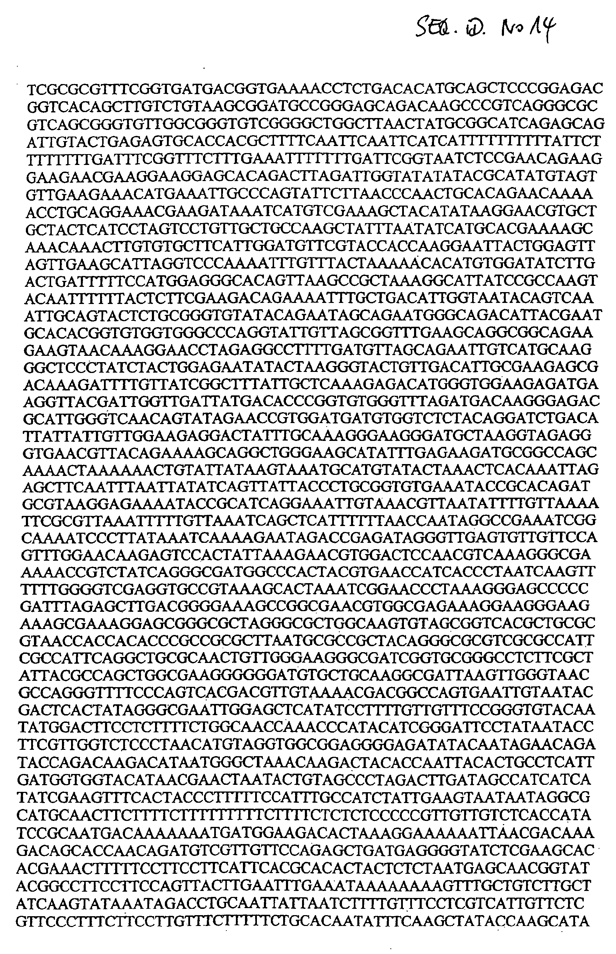

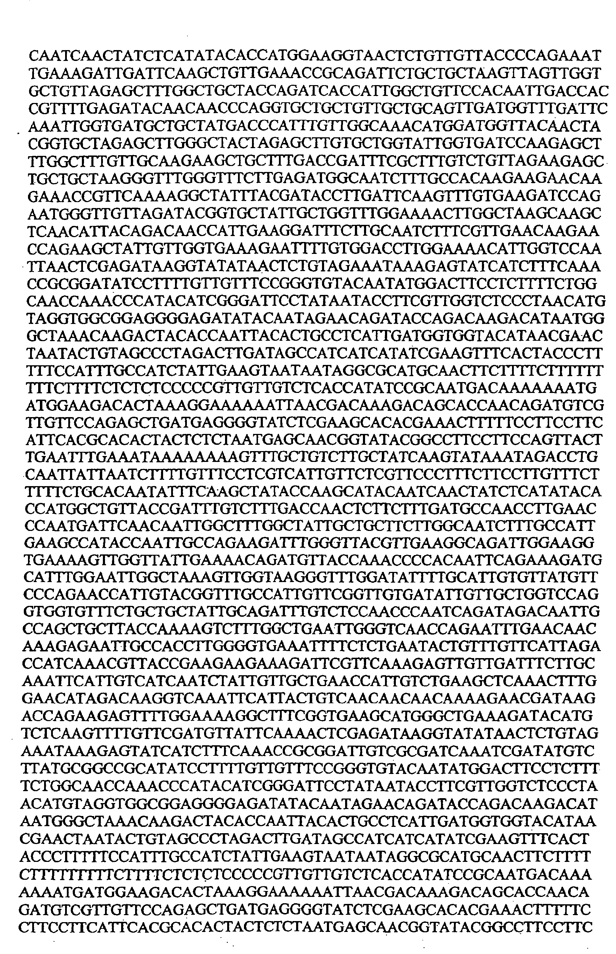

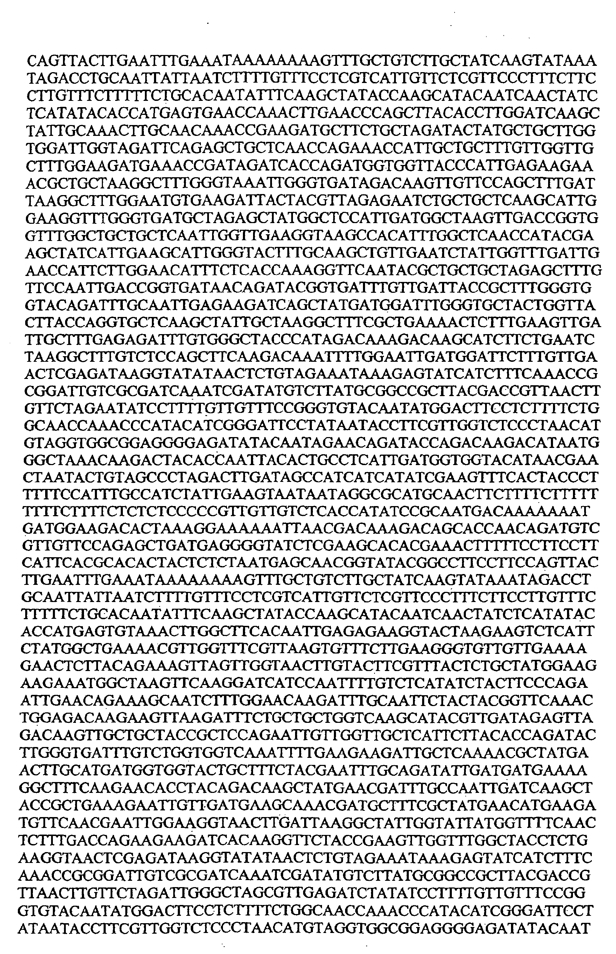

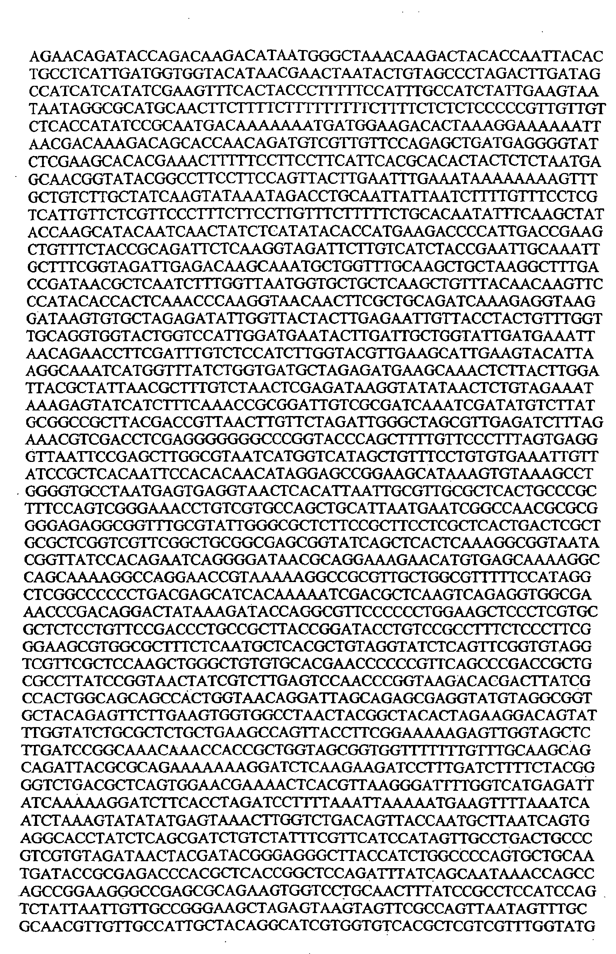

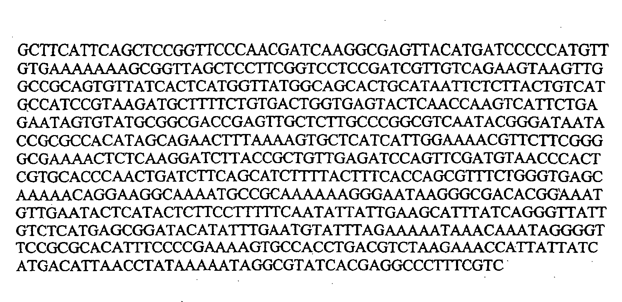

Das Spektrum (Fig. 8) des erfindungsgemäßen Holo-CpcA wurde in folgendem Puffer aufgenommen:

### Lysepuffer:

50 mM NaH₂PO₄
300 mM NaCl
10 mM Imidazol
pH 8,0

### Elutionspuffer:

50 mM NaH₂PO₄
300 mM NaCl
250 mM Imidazol
pH 8,0

Der Lysepuffer und der Elutionspuffer lagen im Verhältnis 60% zu 40% vor.

### 7. Hefestamm und Kultivierungsbedingungen

Als Wirtsorganismus für die erfindungsgemäße heterologe Expression der PC-Gene wurde eine Uracil defiziente Variante des Y190 Stamms der Hefe verwendet. Der Hefestamm Y 190 zeigte ursprünglich folgenden Genotyp: MATa, leu2-3, leu2-112, ura3-52, trp1-901, his3-Δ200, ade2-101, GAL4-Δgal80-Δ, URA3::GAL-LacZ, LYS::GAL-HIS3, cyh^{r}.

Zur Bereitstellung einer Uracil defizienten Y190 Hefe wurden aus diesem Stamm nach der Methode von Boeke *et al.* [Boeke, J., Lacroute, F. and Fink, G. R (1984). A positive selection for mutants lacking orotidine-5-phosphate decarboxylase activity in yeast: 5-Fluoro-orotic acid resistance. Mol. Gen. Genet 197, 345-346] Hefezellen selektiert, die eine Deletion des URA3-Gens aufweisen.

Diese URA3 Deletionsmutanten wurden auf YPAD-Medium oder auf beliebigen synthetischen Komplett-Medien kultiviert. Es hat sich als besonders vorteilhaft erwiesen, anstelle der in der Literatur vorgeschlagenen 2 %-igen Glucose-Konzentration eine bis zu 13 %-ige Glucosekonzentration zu verwenden. Es sind aber alle Konzentrationen zwischen 2 und 20 % möglich.

## Patentansprüche

**1.** Rekombinante Eukaryontenzelle synthetisierend funktionell aktives Phycocyanin oder funktionell aktive Teile daraus.

**2.** Rekombinante Eukaryontenzelle synthetisierend Holo-CpcA und/oder Holo-CpcB.

**3.** Rekombinante Eukaryontenzelle nach Anspruch 1 oder 2 exprimierend die heterodimere PCB Lyase sowie das Apo-CpcA und/oder das Apo-CpcB.

**4.** Rekombinante Eukaryontenzelle nach Anspruch 3 exprimierend des weiteren eine heterologe Hämogygenase und/oder eine heterologe Ferredoxin Oxidoreduktase.

**5.** Rekombinante Eukaryontenzelle nach einem der Ansprüche 3 oder 4, **gekennzeichnet durch** eines oder mehrere der Gene hox 1, pcyA, cpcE, cpcF und cpcA.

**6.** Rekombinante Eukaryontenzelle nach Anspruch 5 transformiert mit einem Vektor umfassend mindestens ein Insert mit einer der folgenden Oligonukleotidsequenzen gemäß einer der SEQ. ID. No. 6 - SEQ. ID. No. 10 oder mit einer dieser Sequenzen komplementären Strang hybridisierenden Oligonukleotiden.

**7.** Rekombinante Eukaryontenzelle nach einem der Ansprüche 1 bis 6, **dadurch gekennzeichnet, dass** mindestens eines der Gene von einem ADH Promotor reguliert wird, der upstream von -700 bp deletiert ist.

**8.** Rekombinante Eukaryontenzelle nach Anspruch 7, **dadurch gekennzeichnet, dass** alle Gene von einem ADH Promotor reguliert werden, der upstream von -700 bp deletiert ist.

**9.** Rekombinante Eukaryontenzelle nach Anspruch 7 oder 8, **gekennzeichnet durch** einen ADH Promotor mit der Oligonukleotidsequenz gemäß SEQ. ID. No. 5 oder mit einer mit dem dazu komplementären Strang hybridisierenden Oligonukleotidsequenz.

**10.** Rekombinante Eukaryontenzelle nach einem der vorherigen Ansprüche, **dadurch gekennzeichnet, dass** sie eine Hefezelle ist.

**11.** Hefezelle mit der DSMZ Aufnahmenummer DSM 16134.

**12.** Vektor umfassend Inserts codierend für eine Hämoxygenase, eine Ferredoxin Oxidoreduktase, eine heterodimäre PCB Lyase und das Apo-CpcA oder Apo-CpcB.

**13.** Vektor nach Anspruch 12, **gekennzeichnet durch** Inserts mit den Genen hox1, pcyA, cpcE, cpcF und cpcA, wobei das cpcA auch **durch** cpcB ersetzt werden kann.

**14.** Vektor nach Anspruch 11, **gekennzeichnet durch** eine oder mehrere der Oligonukleotide gemäß einer der SEQ. ID. No. 6 - SEQ. ID. No. 10 oder mit einer dieser Sequenzen komplementären Strang hybridisierenden Oligonukleotiden.

**15.** Vektor nach einem der Ansprüche 12 bis 14, **dadurch gekennzeichnet, dass** mindestens eines der Gene von einem ADH Promotor reguliert wird, der upstream von -700 bp deletiert ist.

**16.** Vektor nach Anspruch 15, **dadurch gekennzeichnet, dass** alle Gene von einem ADH Promotor reguliert werden, der upstream von -700 bp deletiert ist.

**18.** Vektor nach Anspruch 15 oder 16, **gekennzeichnet durch** einen ADH Promotor mit der Oligonukleotidsequenz gemäß SEQ. ID. No. 5 oder mit einer mit dem dazu komplementären Strang hybridisierenden Oligonukleotidsequenz.

**19.** Vektor mit einer Nukleotidsequenz gemäß SEQ. ID. No. 14.

**20.** Verfahren zur Herstellung eines Phycocyanins oder eines funktionell aktiven Teils davon, insbesondere Holo-CpcA oder Holo-CpcB umfassend folgende Schritte:
- Transformation einer eukaryontischen Wirtszelle mindestens mit cpcA oder cpcB;
- ggf. Addition eines Phycocyanobilins (PCB) und/oder der PCB Lyase
- isolierung des Phycocyanins, des Holo-CpcA und/oder des Holo-CpcB aus der Wirtszelle.

**21.** Verfahren nach Anspruch 20, **dadurch gekennzeichnet, dass** zusätzlich auch eines oder mehrere der Gene hox1, pcyA, cpcE und/oder cpcF rekombinant exprimiert werden.

**22.** Verfahren nach einem der Ansprüche 20 oder 21, **dadurch gekennzeichnet, dass** die endogene Hämkonzentration in der Wirtszelle erhöht wird.

**23.** Verfahren nach Anspruch 20 bis 22, **gekennzeichnet durch** die Stimulierung des endogenen Hämstoffwechsels in der Wirtszelle.

**24.** Verfahren nach Anspruch 23, **gekennzeichnet durch** die Verwendung einer nicht von der Wirtszelle fermentierbaren Kohlenstoffquelle.

**25.** Verfahren nach einem der Ansprüche 20 bis 22, **gekennzeichnet durch** eine Inhibierung des endogenen Hämabbaus in der Wirtszelle.

**25.** Verfahren nach einem der Ansprüche 20 bis 25, **gekennzeichnet durch** die Erhöhung der Gukosekonzentration im Medium der Wirtszelle auf eine Konzentration, die die Aktivität des ADH-Promotors stimuliert.

**26.** Verfahren nach Anspruch 25, **gekennzeichnet durch** die Gabe von mindestens 13 % Glucose.

**27.** Verfahren nach einem der Ansprüche 20 bis 26, **gekennzeichnet durch** eine 2 %-ige Ethanolkonzentration im Kulturmedium der Wirtszelle.

**28.** Verfahren nach einem der Ansprüche 20 bis 27, **gekennzeichnet durch** eine Wirtszelle nach einem der Ansprüche 1 bis 11.

**29.** Verfahren nach einem der Ansprüche 20 bis 26, **dadurch gekennzeichnet, dass** die Transformation mit Hilfe eines Vektors nach einem der Ansprüche 12 bis 19 erfolgt.

**30.** Rekombinantes Phycocyanin oder funktionell aktive Teile daraus, hergestellt nach einem der Ansprüche 20 bis 29.

**31.** Rekombinantes Phycocyanin oder funktionell aktive Teile daraus nach Anspruch 30, **gekennzeichnet durch** eine Glykosylierung.

**32.** Rekombinantes Phycocyanin oder funktionell aktive Teile daraus nach einem der Ansprüche 30 oder 31, **dadurch gekennzeichnet, dass** die Glykosylierung an mindestens einer der Position 6, 10 oder 162 der Aminosäuresequenz vorliegt.

**33.** Rekombinantes Phycocyanin oder funktionell aktive Teile daraus nach einem der Ansprüche 30 bis 32, **dadurch gekennzeichnet, dass** das Emissionsmaximum im Vergleich zum nativen Phycocyanin, insbesondere zum native Holo-CpcA bei pH 8 in den längerwelligen Bereich verschoben ist.

**34.** Rekombinantes Holo-CpcA mit einem Emissionsmaximum bei pH 8 von 643 nm.

**35.** Fusionsprotein enthaltend ein rekombinantes Phycocyanin oder funktionell aktive Teile davon, insbesondere rekombinantes Holo-CpcA, nach einem der Ansprüche 30 bis 34.

**36.** Antikörper oder Fragmente davon, **dadurch gekennzeichnet, dass** er spezifisch ein rekombinantes Phycocyanin nach einem der Ansprüche 30 bis 34 erkennt.

**37.** Antikörper oder Fragmente davon, **dadurch gekennzeichnet, dass** er spezifisch funktionell aktive Teile des rekombinantes Phycocyanins nach einem der Ansprüche 30 bis 34, insbesondere rekombinantes Holo-CpcA, erkennt.
